# EUROPÄISCHE PATENTSCHRIFT

(11) **EP 4 534 999 B1**
(45) Veröffentlichungstag und Bekanntmachung des Hinweises auf die Patenterteilung: **11.11.2026**
(21) Anmeldenummer: 24201240.9
(22) Anmeldetag: 19.09.2024
(51) Int. Cl.: G01N 33/497, A61B 5/097

(54) **GASMESSGERÄT UND GASMESSVERFAHREN**
GAS MEASURING DEVICE AND GAS MEASURING METHOD
APPAREIL ET PROCÉDÉ DE MESURE DE GAZ

(30) Priorität: 26.09.2023 DE 102023125988
(43) Veröffentlichungstag der Anmeldung: 09.04.2025
(73) Patentinhaber: Dräger Safety AG & Co. KGaA, 23560 Lübeck (DE)
(72) Erfinder: Baesler, Malte, 23558 Lübeck (DE); Rekow, Jens, 23558 Lübeck (DE); Reier, Tobias, 23558 Lübeck (DE); Brunswick, Franco Luis, 23558 Lübeck (DE); Kahns, Peter, 23558 Lübeck (DE)

(56) Entgegenhaltungen:
- WO-A1-2021/250674
- DE-A1- 19 941 586
- GB-A- 2 592 708
- KR-Y1- 200 357 468
- US-A1- 2021 065 901

## Beschreibung

Die Erfindung betrifft ein Gasmessgerät und ein Gasmessverfahren, welche die Konzentration eines Bestandteils in einem Gasgemisch zu messen vermögen.

Die Aufgabe, die Konzentration eines Bestandteils in einem Gasgemisch zu messen, tritt beispielsweise dann auf, wenn ein Proband darauf untersucht werden soll, ob er Alkohol zu sich genommen hat. Falls er Alkohol zu sich genommen hat, enthält die von ihm ausgeatmete Luft Atemalkohol. Bei dieser Anwendung ist das Gasgemisch eine Atemprobe, die der Proband abgegeben hat, und der Bestandteil, auf den das Gasgemisch untersucht werden soll, ist Atemalkohol. Der Bestandteil kann auch eine andere Substanz sein, die sich in einer Atemprobe nachweisen lässt.

US 2021 / 0 065 901 A1 offenbart ein Gasmessgerät, das eine Eingabeeinheit mit Ein- und Auslassöffnung, einen Grundkörper, eine Zuführeinheit, eine Messkammer im Inneren des Grundkörpers, eine Abführeinheit und einen oder mehrere Gassensoren umfasst. Das Gerät ist dafür ausgestaltet, ein Gasgemisch durch die Eingabeeinheit fließen zu lassen und dabei an einem Eintrittspunkt eine Gasprobe abzuzweigen, sodass diese durch die Zuführeinheit in die Messkammer fließt und anschließend aus dieser heraus durch die Abführeinheit, wobei sie an einem vom Eintrittspunkt beabstandeten Austrittspunkt wieder in die Eingabeeinheit hineinfließt.

WO 2021 / 250674 A1 offenbart ein Gasmessgerät, umfassend eine Eingabeeinheit, einen Grundkörper, eine Zuführeinheit und eine Abführeinheit. Das Gasgemisch fließt durch die Einlassöffnung in die Eingabeeinheit hinein und hindurch, wobei eine Gasprobe durch die Zuführeinheit abgezweigt und in eine Messkammer geleitet wird. Aus der Messkammer fließt sie dann durch die Abführeinheit zum Austrittspunkt aus dem Grundkörper heraus, wobei die abgezweigte Gasprobe und der Rest des Gasgemischs an zwei voneinander beabstandeten Stellen in die Umgebung des Gasmessgeräts fließen.

Der Erfindung liegt die Aufgabe zugrunde, ein Gasmessgerät und ein Gasmessverfahren bereitzustellen, welche eine Messkammer zur Aufnahme einer Gasprobe umfassen und mit größerer Zuverlässigkeit als bekannte Geräte und Verfahren eine Gasprobe zur Messkammer hin- und von der Messkammer wegzuleiten vermögen.

Die Aufgabe wird durch ein Gasmessgerät mit den Merkmalen des Anspruchs 1 und durch ein Gasmessverfahren mit den Merkmalen des Anspruchs 11 gelöst. Vorteilhafte Ausgestaltungen des erfindungsgemäßen Gasmessgeräts sind, soweit sinnvoll, auch vorteilhafte Ausgestaltungen des erfindungsgemäßen Gasmessverfahrens und umgekehrt.

Nachfolgend werden die Begriffe "Fluidführungseinheit" und "Fluidfördereinheit" verwendet. Eine Fluidführungseinheit ist ein Bauteil, das ein Fluid entlang einer Trajektorie zu leiten vermag, wobei die Trajektorie durch die Geometrie, Konstruktion und / oder Anordnung der Fluidführungseinheit vorgegeben ist. Idealerweise verhindert die Fluidführungseinheit, dass das geleitete Fluid diese Trajektorie verlässt. Ein Schlauch und eine Röhre sind zwei Beispiele für eine Fluidführungseinheit. Eine Fluidfördereinheit vermag ein Fluid zu fördern, also zu bewegen, insbesondere durch eine Fluidführungseinheit hindurch. Eine Pumpe, ein Gebläse und eine Kolben-Zylinder-Einheit sind Beispiele für eine Fluidfördereinheit.

Das erfindungsgemäße Gasmessgerät und das erfindungsgemäße Gasmessverfahren vermögen die Konzentration mindestens eines Bestandteils in einem Gasgemisch zu messen. Das Gasgemisch ist beispielsweise Atemluft, die ein Proband abgegeben hat, und der gesuchte Bestandteil ist Atemalkohol oder eine andere Substanz, die in einer Atemprobe enthalten sein kann und die detektiert werden soll. Das Gasgemisch kann auch aus einer anderen Quelle als aus dem Mund eines Probanden stammen.

Das erfindungsgemäße Gasmessgerät umfasst eine röhrenförmige Eingabeeinheit, wobei die Eingabeeinheit eine Einlassöffnung und eine Auslassöffnung aufweist. Bevorzugt erstreckt die Eingabeeinheit sich entlang einer Längsachse. Möglich ist, dass die Eingabeeinheit sich von der Einlassöffnung auf die Auslassöffnung zu verjüngt.

Weiterhin umfasst das Gasmessgerät einen Grundkörper. Die Eingabeeinheit ist mit dem Grundkörper verbunden oder lässt sich mit dem Grundkörper verbinden, bevorzugt lösbar verbinden. Eine lösbare Verbindung ermöglicht es, die Eingabeeinheit nach der Eingabe einer Gasprobe auszuwechseln und den Grundkörper weiter zu verwenden.

**Im** Inneren des Grundkörpers sind mindestens eine Messkammer und mindestens ein Gassensor des Gasmessgeräts angeordnet, optional mehrere Gassensoren, in einer Ausgestaltung mehrere Messkammern und mehrere Gassensoren. Nachfolgend wird eine Ausgestaltung mit einer Messkammer und einem Gassensor beschrieben. Diese Beschreibung gilt entsprechend auch für eine Ausgestaltung mit mehreren Gassensoren und / oder mehreren Messkammern.

Die Messkammer vermag eine Gasprobe aufzunehmen. Der Grundkörper weist einen Eintrittspunkt und einen Austrittspunkt auf, wobei diese beiden Punkte voneinander beabstandet sind. Eine Zuführ-Fluidführungseinheit verbindet den Eintrittspunkt mit der Messkammer. Eine Abführ-Fluidführungseinheit verbindet die Messkammer mit dem Austrittspunkt. Diese beiden Fluidführungseinheiten sind voneinander beabstandet und kreuzungsfrei im Grundkörper angeordnet. Bevorzugt erstrecken sich diese beiden Fluidführungseinheiten oder wenigstens jeweils ein Segment dieser beiden Fluidführungseinheiten entlang zweier Längsachsen, wobei die beiden Längsachsen jeweils einen Winkel von mindestens 60° mit der Längsachse der Eingabeeinheit einschließen und besonders bevorzugt beide senkrecht auf der Längsachse der Eingabeeinheit stehen.

Das erfindungsgemäße Gasmessverfahren wird unter Verwendung eines derartigen Gasmessgeräts durchgeführt. Das Gasmessgerät ist dazu ausgestaltet, die folgenden Schritte durchzuführen, und das Gasmessverfahren umfasst dementsprechend die folgenden Schritte:
- Die Eingabeeinheit ist oder wird mit dem Grundkörper verbunden.
- Ein Gasgemisch, z.B. eine Atemprobe, fließt durch die Einlassöffnung hindurch in die Eingabeeinheit.
- Im Eintrittspunkt wird eine Gasprobe aus dem durchfließenden Gasgemisch abgezweigt, also entnommen. Der Rest des Gasgemischs, also das Gasgemisch ohne die abgezweigte Gasprobe, fließt am Eintrittspunkt und anschließend am Austrittspunkt vorbei und verlässt dann die Eingabeeinheit durch die Auslassöffnung. Bevorzugt werden höchstens 10 Vol.-% des Gasgemischs als Gasprobe abgezweigt.
- Die abgezweigte Gasprobe fließt durch die Zuführ-Fluidführungseinheit hindurch in die Messkammer.
- Die abgezweigte Gasprobe fließt aus der Messkammer heraus und durch die Abführ-Fluidführungseinheit hindurch zum Austrittspunkt und dort aus dem Grundkörper heraus.

Erfindungsgemäß ist das Gasmessgerät wie folgt ausgestaltet, und das Gasmessverfahren umfasst die folgenden Schritte:
- Im Austrittspunkt fließt die abgezweigte Gasprobe aus dem Grundkörper heraus und nach dem Austritt aus dem Grundkörper an der Eingabeeinheit vorbei und dann in eine Umgebung des Gasmessgeräts.
- Während die abgezweigte Gasprobe an der Eingabeeinheit vorbeifließt, fließt sie in eine Richtung weg von der Einlassöffnung und bevorzugt in eine Richtung parallel zu einer Längsachse der Eingabeeinheit und damit parallel zum Rest des Gasgemischs, also zum nicht abgezweigten Teil des Gasgemischs.

Sowohl die abgezweigte Gasprobe als auch der Rest des Gasgemischs fließen wieder in eine Umgebung des Gasmessgeräts.

Der oder jeder Gassensor ist dazu ausgestaltet, die Konzentration des Bestandteils in der Gasprobe zu messen, während die Gasprobe sich in der Messkammer befindet. Genauer gesagt: Der oder jeder Gassensor vermag jeweils eine physikalische Größe zu messen, die mit der Konzentration des Bestandteils korreliert. Im Falle von mindestens zwei Gassensoren messen diese beiden Gassensoren bevorzugt zwei verschiedene Größen, die beide mit der Konzentration des Bestandteils korrelieren.

Nachfolgend werden die Begriffe "flussaufwärts" und "flussabwärts" sowie "links" und "rechts" verwendet. Diese Begriffe beziehen sich auf die Richtung, in der ein Gasgemisch durch die Eingabeeinheit hindurchfließt, und haben in Bezug auf diese Richtung die üblichen Bedeutungen. Die Begriffe "oben" und "unten" beziehen sich auf eine Orientierung, in der ein Benutzer den Grundkörper in einer Hand hält und die Eingabeeinheit sich oberhalb des Grundkörpers befindet. Bei dieser Anwendung bezeichnet "flussabwärts" eine Richtung weg vom Benutzer.

Erfindungsgemäß fließt das Gasgemisch durch die Einlassöffnung hindurch in die röhrenförmige Eingabeeinheit. Wenigstens derjenige Teil des Gasgemischs, der nicht im Eintrittspunkt abgezweigt wird, verlässt die Eingabeeinheit durch die Auslassöffnung hindurch. Der abgezweigte Teil, also die Gasprobe, verlässt den Grundkörper durch den Austrittspunkt hindurch. Erfindungsgemäß fließt der abgezweigte Teil an der Eingabeeinheit vorbei und in eine Richtung weg von der Einlassöffnung der Eingabeeinheit.

In vielen Fällen wird das Gasmessgerät von einem Menschen gehalten, beispielsweise weil dieser Mensch oder ein anderer Mensch das Gasgemisch in die Eingabeeinheit eingibt. Das gerade beschriebene Merkmal verringert in Verbindung mit der röhrenförmigen Gestalt der Eingabeeinheit das Risiko, dass das Gasgemisch einem Menschen ins Gesicht geblasen wird oder ihm ins Gesicht strömt oder auf andere Weise ungewollt einen Menschen erreicht, nachdem das Gasgemisch durch die Eingabeeinheit geflossen ist. Der Grundkörper lässt sich so ausrichten, dass die Auslassöffnung der Eingabeeinheit nicht auf einen Menschen zeigt. Das unerwünschte Ereignis, dass das Gasgemisch einem Menschen ins Gesicht strömt, lässt sich verhindern, ohne dass notwendigerweise in der Eingabeeinheit ein Rückhalteelement vorhanden ist.

Das Ereignis, dass das austretende Gasgemisch einen Menschen erreicht, ist unerwünscht, und zwar insbesondere deshalb, weil das Gasgemisch Krankheitserreger enthalten kann, was vor allem dann möglich ist, wenn das Gasgemisch ausgeatmete Atemluft ist. Deshalb wird gemäß der Erfindung das Gasgemisch auf eine definierte Weise vom Gasmessgerät weggeführt.

Die Eingabeeinheit umfasst neben der Einlassöffnung und der Auslassöffnung eine weitere Öffnung, wobei diese weitere Öffnung als der Eintrittspunkt fungiert, in dem die Gasprobe abgezweigt wird. Die Erfindung vermeidet die Notwendigkeit, außer dem Eintrittspunkt eine zusätzliche Öffnung in der Wand der Eingabeeinheit anzubringen, wobei diese zusätzliche Öffnung als der Austrittspunkt fungiert. Zwei Öffnungen in einer Wand der Eingabeeinheit führen in manchen Fällen zu mehr Verwirbelungen und Turbulenzen in der Eingabeeinheit als nur eine Öffnung.

In der Regel ändert die abgezweigte Gasprobe ihre Fließrichtung, wenn die Gasprobe aus der Eingabeeinheit abgezweigt wird und durch die Zuführ-Fluidführungseinheit fließt. Dieses Merkmal reduziert die Gefahr, dass Substanzen in die Messkammer gelangen, welche für die Messkammer oder für den Gassensor schädlich sind. Insbesondere werden die Gefahren reduziert, dass Wasser in der Gasprobe auf einer Oberfläche der Messkammer oder einer Oberfläche eines Sensors kondensiert oder dass größere Partikel aus der Umgebung oder der Eingabeeinheit in die Messkammer gelangen. Diese Gefahr wäre größer, wenn das Gasgemisch ohne Richtungsänderung von der Einlassöffnung in die Messkammer fließen würde.

Erfindungsgemäß fließt die abgezweigte Gasprobe durch die Zuführ-Fluidführungseinheit hindurch in die Messkammer hinein und durch die Abführ-Fluidführungseinheit hindurch aus der Messkammer heraus. Die Erfindung erspart die Notwendigkeit, dass eine Gasprobe durch dieselbe Fluidführungseinheit zuerst in die Messkammer hinein und anschließend aus der Messkammer herausfließt. Die Ausgestaltung mit einer einzigen Fluidführungseinheit oder mit zwei sich kreuzenden Fluidführungseinheiten kann zu einem Rückstau in der einzigen Fluidführungseinheit oder am Kreuzungspunkt führen. Falls die Gasprobe von einem Kolben angesaugt wird, so muss dieser Kolben bei einer einzigen Fluidführungseinheit in vielen Fällen einen relativ langen Hub ausführen, also einen relativ langen Weg zurücklegen. Die Erfindung ermöglicht es, die Gasprobe mithilfe einer relativ kleinen Fluidfördereinheit abzuzweigen und dann wieder aus der Messkammer auszustoßen. Eine kontinuierliche Messung, die theoretisch zeitlich unbefristet ist, wird ermöglicht. Bei Verwendung eines Kolbens in Verbindung mit einer einzigen Fluidführungseinheit müsste die Messung zwangsläufig häufig unterbrochen werden.

Erfindungsgemäß fließt die Gasprobe aus der Messkammer durch die Abführ-Fluidführungseinheit hindurch zum Austrittspunkt und tritt im Austrittspunkt aus der Abführ-Fluidführungseinheit aus.

Die abgezweigte Gasprobe und der Rest des Gasgemischs treten an zwei voneinander beabstandeten Stellen in die Umgebung aus. Gemäß einer Ausgestaltung vermischt sich die Gasprobe in einem Vermischungsbereich, der flussabwärts von der Auslassöffnung der Eingabeeinheit angeordnet ist, mit demjenigen Teil des Gasgemischs, der durch die Eingabeeinheit geflossen ist, ohne abgezweigt zu werden.

Gemäß der Erfindung wird dann, wenn die Eingabeeinheit mit dem Grundkörper verbunden ist, zwischen der Eingabeeinheit und dem Grundkörper ein Kanal gebildet. Dieser Kanal verbindet den Austrittspunkt mit dem Vermischungsbereich. Der Vermischungsbereich gehört zu einer Umgebung des Gasmessgeräts oder steht in einer Fluidverbindung mit der Umgebung. Bevorzugt erstreckt sich der Kanal parallel zu einer Längsachse der röhrenförmigen Eingabeeinheit. Die Gasprobe fließt aus der Messkammer durch die Abführ-Fluidführungseinheit zum Austrittspunkt und anschließend vom Austrittspunkt durch diesen Kanal hindurch und damit an der Eingabeeinheit vorbei zum Vermischungsbereich.

In manchen Fällen reduzieren die Erfindung und insbesondere die gerade beschriebene Ausgestaltung die Gefahr, dass in der Eingabeeinheit unerwünschte Verwirbelungen auftreten und / oder dass es in der Abführ-Fluidführungseinheit zu einem Rückstau kommt. Der Kanal leitet mit besonders hoher Zuverlässigkeit das Gasgemisch weg von der Einlassöffnung der Eingabeeinheit. Damit wird die Gefahr weiter verringert, dass ein Teil des Gasgemischs einem Menschen, vor dessen Gesicht sich die Eingabeeinheit befindet, ins Gesicht fließt.

Möglich ist, dass die Auslassöffnung der Eingabeeinheit bündig mit dem Grundkörper abschließt. Möglich ist auch, dass die Eingabeeinheit flussaufwärts und / oder flussabwärts über den Grundkörper übersteht. In einer Ausgestaltung steht hingegen umgekehrt der Grundkörper flussabwärts von der Eingabeeinheit über die Eingabeeinheit über. Flussabwärts von der Auslassöffnung wird ein Bereich gebildet, der auf einer Seite von der Eingabeeinheit, insbesondere von der Auslassöffnung, und auf einer anderen Seite vom flussabwärts überstehenden Teil des Grundkörpers gebildet und begrenzt wird. In diesen Bereich gelangt die gesamte Menge des Gasgemischs. Die abgezweigte Gasprobe gelangt durch den Austrittspunkt hindurch und an der Eingabeeinheit vorbei in diesen Bereich, und der Rest des Gasgemischs gelangt durch die Auslassöffnung hindurch in diesen Bereich. Diese Ausgestaltung lässt sich mit dem gerade beschriebenen Merkmal, dass ein Kanal zwischen dem Grundkörper und der Eingabeeinheit zum Vermischungsbereich führt, kombinieren.

In einer Ausgestaltung unterteilt ein Trennelement die Eingabeeinheit in eine einlassseitige Kammer und eine auslassseitige Kammer. Die Einlassöffnung grenzt an die einlassseitige Kammer an, die Auslassöffnung an die auslassseitige Kammer. In dieses Trennelement ist mindestens eine Durchlassöffnung eingelassen. Sowohl der Eintrittspunkt als auch der Austrittspunkt grenzen an die auslassseitige Kammer an. Das Trennelement trennt die auslassseitige Kammer von der Einlassöffnung. Die gesamte Menge des Gasgemischs fließt durch die Einlassöffnung und durch die einlassseitige Kammer hindurch bis zum Trennelement. Wenigstens ein Teil des Gasgemischs fließt durch die Durchlassöffnung im Trennelement hindurch in die auslassseitige Kammer hinein.

Die Gasprobe wird also flussabwärts vom Trennelement und in der auslassseitigen Kammer aus der Eingabeeinheit abgezweigt. Diese Ausgestaltung reduziert - verglichen mit einer Ausgestaltung ohne ein Trennelement in der Eingabeeinheit - die Gefahr, dass in der auslassseitigen Kammer Verwirbelungen oder auch ein sehr großer Überdruck oder Unterdruck relativ zum Umgebungsdruck auftreten. Dadurch wird es erleichtert, eine Gasprobe mit einem relativ gleichmäßigen Volumenfluss abzuzweigen.

Das Trennelement kann als ein rein passives mechanisches Bauteil ausgestaltet sein. In einer Realisierungsform erstreckt sich die einlassseitige Kammer entlang der gesamten Länge der Eingabeeinheit, also von der Einlassöffnung zur Auslassöffnung. Gesehen in eine Fließrichtung des Gasgemischs durch die Eingabeeinheit hindurch befindet sich ein Segment der einlassseitigen Kammer neben oder über der auslassseitigen Kammer. Diese Realisierungsform verringert weiter das Risiko, dass in der auslassseitigen Kammer Verwirbelungen oder ein großer Unterdruck oder Überdruck auftreten.

Bevorzugt lässt sich die Eingabeeinheit lösbar mit dem Grundkörper verbinden und wieder vom Grundkörper trennen. In einer Ausgestaltung umfasst das Gasmessgerät einen Drucksensor. Dieser Drucksensor vermag den Druck in einer Fluidführungseinheit zu messen - genauer gesagt: direkt den Druck oder eine Größe, die mit dem Druck korreliert. In einer Realisierungsform ist diese Fluidführungseinheit die Zuführ-Fluidführungseinheit, in einer anderen Realisierungsform die Abführ-Fluidführungseinheit. Abhängig vom gemessenen Druck vermag das Gasmessgerät - genauer gesagt: ein signalverarbeitendes Steuergerät oder sonstiges Gerät des Gasmessgeräts - automatisch zu entscheiden, ob die Eingabeeinheit mit dem Grundkörper verbunden ist oder nicht. Bei aufgesetzter Eingabeeinheit ist der Druck in der Regel signifikant größer als bei nicht aufgesetzter Eingabeeinheit, liegt also oberhalb einer vorgegebenen Druckdifferenz-Schranke. Dieser signifikante Unterschied tritt mindestens auf, während ein Fluid durch die Fluidführungseinheit fließt, insbesondere während ein Gasgemisch in die Eingabeeinheit eingegeben wird oder während die Messkammer ausgespült wird.

Diese Ausgestaltung reduziert die Gefahr, dass eines der folgenden unerwünschten Ereignisse auftritt, ohne detektiert zu werden:
- Gemäß der Ausgestaltung vermag der Drucksensor den Druck in einer Fluidführungseinheit zu messen. Umgebungsluft fließt durch diese Fluidführungseinheit in die Messkammer oder aus der Messkammer. Dadurch wird die Messkammer nach einer Messung ausgespült und steht nach dem Ausspülen für eine neue Messung zur Verfügung. Dieses Ausspülen ist ein gewünschter Vorgang. Beim Ausspülen sollte eine Eingabeeinheit nicht mit dem Grundkörper verbunden sein. Nur ohne Eingabeeinheit auf dem Grundkörper wird die Messkammer tatsächlich vollständig ausgespült.
- Obwohl das Gasmessgerät jetzt dafür benutzt werden soll, ein Gasgemisch auf den Bestandteil zu untersuchen, ist keine Eingabeeinheit mit dem Grundkörper verbunden. Dies ist offensichtlich ein Fehler.

Bevorzugt generiert das Gasmessgerät eine Meldung, wenn es eines dieser beiden unerwünschten Ereignisse detektiert hat. Diese Meldung wird in einer von einem Menschen wahrnehmbaren Form ausgegeben, und zwar bevorzugt von einer Ausgabeeinheit des Gasmessgeräts.

Erfindungsgemäß wird ein Teil des Gasgemischs, das durch die Eingabeeinheit fließt, abgezweigt und als Gasprobe durch die Zuführ-Fluidführungseinheit hindurch in die Messkammer geleitet. In einer Ausgestaltung reicht die kinetische und / oder die potenzielle Energie des Gasgemischs, das in die Eingabeeinheit hineinfließt, aus, um das Gasgemisch abzuzweigen. Dies gilt beispielsweise dann, wenn das Gasgemisch eine Atemprobe ist, die ein Proband abgibt, vorausgesetzt der Proband atmet ausreichend stark aus. Möglich ist, dass das Gasmessgerät keine eigene Fluidfördereinheit umfasst.

In einer Ausgestaltung umfasst das Gasmessgerät hingegen eine Fluidfördereinheit, beispielsweise eine Pumpe oder eine Kolben-Zylinder-Einheit oder ein Gebläse. Die Fluidfördereinheit lässt sich einschalten und ausschalten. Die Fluidfördereinheit vermag zumindest die Gasprobe aus der Messkammer heraus und durch die Abführ-Fluidführungseinheit hindurch zu fördern. In der Regel fließt dann Gas durch die Zuführ-Fluidführungseinheit hindurch in die Messkammer, insbesondere deshalb, weil ein Unterdruck in der Messkammer erzeugt wird. Dadurch wird die Messkammer ausgespült, und eine neue Gasprobe wird in die Messkammer gesaugt. In einer Realisierungsform trägt die Fluidfördereinheit außerdem dazu bei, oder sie bewirkt sogar ausschließlich, dass die Gasprobe von dem Gasgemisch, das durch die Eingabeeinheit fließt, abgezweigt wird. Bei dieser Realisierungsform saugt bevorzugt die Fluidfördereinheit die Gasprobe durch die Zuführ-Fluidführungseinheit hindurch in die Messkammer hinein.

Die Ausgestaltung mit der Fluidfördereinheit lässt sich mit der Ausgestaltung, bei der ein Drucksensor ein Maß für den Druck in einer Fluidführungseinheit misst, kombinieren. Bei dieser Kombination misst der Drucksensor ein Maß für den Druck in der Abführ-Fluidführungseinheit. Aufgrund des gemessenen Drucks, also aufgrund eines Signals des Drucksensors, vermag das Gasmessgerät automatisch zu entscheiden, ob die Fluidfördereinheit eingeschaltet oder ausgeschaltet ist. In der Regel soll die Fluidfördereinheit wenigstens zeitweise eingeschaltet sein, wenn ein Gasgemisch in die Eingabeeinheit eingegeben wird.

Die gerade beschriebene Ausgestaltung lässt sich mit der Ausgestaltung kombinieren, bei der die Eingabeeinheit sich lösbar mit dem Grundkörper verbinden und wieder vom Grundkörper trennen lässt. Gemäß dieser Kombination vermag das Gasmessgerät automatisch folgende drei Situationen voneinander zu unterscheiden:
- Die Fluidfördereinheit ist ausgeschaltet (niedriger Druck).
- Die Fluidfördereinheit ist eingeschaltet, und die Eingabeeinheit ist nicht mit dem Grundkörper verbunden (mittlerer Druck).
- Die Fluidfördereinheit ist eingeschaltet, und die Eingabeeinheit ist mit dem Grundkörper verbunden (hoher Druck).

In manchen Fällen vermag das Gasmessgerät außerdem folgende unerwünschte Situation zu detektieren: Die Fluidfördereinheit ist ausgeschaltet, aber ein Druck zwischen dem niedrigen Druck und dem mittleren Druck ist in der Abführ-Fluidführungseinheit vorhanden. Dies kann ein Indiz dafür sein, dass ein Proband eine Atemprobe in die Eingabeeinheit eingibt, obwohl die Fluidfördereinheit ausgeschaltet ist. Dies ist oft unerwünscht.

In einer anderen Ausgestaltung verwendet das Gasmessgerät das Signal des Drucksensors, um ein Maß für einen Volumenfluss zu messen, wobei die Fluidfördereinheit diesen Volumenfluss erzielt. Der Volumenfluss ist das Volumen pro Zeiteinheit, das durch eine Fluidführungseinheit fließt. Die Kenntnis des Volumenflusses lässt sich beispielsweise dafür verwenden, um die Fluidfördereinheit mit dem Regelungsziel zu regeln, dass der tatsächliche und in der Regel zeitlich veränderliche Volumenfluss einem vorgegebenen zeitlichen Verlauf folgt, insbesondere dass der Volumenfluss zeitlich konstant sein soll. Die beiden Ausgestaltungen,
- dass das Signal des Drucksensors verwendet wird, um die drei Situationen mit der lösbaren Eingabeeinheit und der ein- und ausschaltbaren Fluidfördereinheit voneinander zu unterscheiden, und
- dass das Signal dazu verwendet wird, um das Maß für den Volumenfluss zu messen,
lassen sich miteinander kombinieren. Mit anderen Worten: Das Signal des Drucksensors lässt sich für die gerade beschriebenen beiden verschiedene Zwecke nutzen.

In einer Ausgestaltung umfasst die gerade beschriebene Fluidfördereinheit folgende Bestandteile:
- eine Kammer, deren Volumen sich verändern lässt,
- einen Stellantrieb oder Motor,
- ein Ansaug-Rückschlagventil und
- ein Ausstoß-Rückschlagventil.

Der Stellantrieb vermag das Volumen der Kammer zu verändern. Beispielsweise wird die Kammer durch eine Membrane oder sonstige bewegliche Wand begrenzt, und der Stellantrieb ist mechanisch mit dieser beweglichen Wand verbunden. Die Kammer kann einen Balg umfassen.

Eine Ansaug-Fluidführungseinheit verbindet die Kammer der Fluidfördereinheit mit der Messkammer. Eine Ausstoß-Fluidführungseinheit verbindet die Kammer mit der Abführ-Fluidführungseinheit. Dadurch steht die Kammer in einer Ansaug-Fluidverbindung mit der Messkammer und in einer Ausstoß-Fluidverbindung mit der Abführ-Fluidführungseinheit. Das Ansaug-Rückschlagventil ermöglicht einen Fluss von Fluid durch die Ansaug-Fluidverbindung hindurch in die Kammer und unterbindet einen Fluss in die entgegengesetzte Richtung. Das Ausstoß-Rückschlagventil ermöglicht einen Fluss von Fluid durch die Ausstoß-Fluidverbindung hindurch in die Abführ-Fluidführungseinheit und unterbindet einen Fluss in die entgegengesetzte Richtung.

Bei dieser Ausgestaltung lässt sich die Fluidfördereinheit sowohl dafür verwenden, um eine Gasprobe aus dem Gasgemisch, das durch die Eingabeeinheit strömt, abzuzweigen und in die Messkammer zu fördern, als auch dafür, die Gasprobe aus der Messkammer auszustoßen und dadurch die Messkammer auszuspülen. In vielen Fällen ermöglicht diese Ausgestaltung es, eine Fluidfördereinheit zu realisieren, die relativ wenig Bauraum benötigt und / oder relativ wenig elektrische Energie verbraucht. Außerdem verhindert diese Anordnung in vielen Fällen, dass Gas in unerwünschter Weise in entgegengesetzter Richtung durch die Messkammer fließt.

Erfindungsgemäß vermag der oder jeder Gassensor des Gasmessgeräts die Konzentration und / oder den Anteil des Bestandteils an der Gasprobe in der Messkammer zu messen. In einer Ausgestaltung ist der oder ein Gassensor als photoelektrischer Sensor ausgestaltet. Der Gassensor umfasst eine Strahlungsquelle und einen Photodetektor. Die Strahlungsquelle vermag elektromagnetische Strahlung zu emittieren. Die emittierte elektromagnetische Strahlung durchdringt die Messkammer und trifft auf den Photodetektor. Der Photodetektor vermag abhängig von der Intensität der auftreffenden Strahlung ein Signal zu generieren, ist also ein optoelektrischer Detektor. Das generierte Signal umfasst eine Information über die Konzentration des Bestandteils der Gasprobe in der Messkammer.

Ein dergestalt ausgestalteter Gassensor vermag kontinuierlich, d. h. mit einer hohen Abtastfrequenz, ein Signal über die aktuelle Konzentration des Bestandteils zu liefern. Außerdem verbraucht ein solcher Sensor keine Chemikalien. Verglichen mit anderen Gassensoren ist ein solcher Sensor in vielen Fällen unempfindlicher gegen Substanzen, die in die Messkammer eindringen, und unempfindlicher gegenüber hohen Temperaturen, die erheblichen Einfluss auf eine Detektionsgröße nehmen können oder zu einer Verdunstung einer Chemikalie führen können. Daher ist es in vielen Fällen nicht erforderlich, die Messkammer fluiddicht gegen die Umgebung abzudichten, wenn das Gasmessgerät nicht verwendet wird.

In einer Fortbildung dieser Ausgestaltung umfasst das Gasmessgerät zusätzlich zum optoelektrischen Sensor einen elektrochemischen Sensor. In einer Alternative ist das Gasmessgerät so ausgestaltet, dass die abgezweigte Gasprobe nacheinander beide Sensoren erreicht. Die beiden Sensoren sind also in Reihe geschaltet. In einer anderen Alternative ist das Gasmessgerät so ausgestaltet, dass eine weitere Gasprobe aus der Eingabeeinheit abgezweigt wird und die weitere Gasprobe den elektrochemischen Sensor erreicht. Oder die abgezweigte Gasprobe wird auf die beiden Sensoren aufgeteilt. Die beiden Sensoren sind also parallel geschaltet.

Der weitere Gassensor weist eine elektrische Detektionsgröße auf. Beim Betrieb läuft im weiteren Gassensor eine chemische Reaktion ab. Die ablaufende chemische Reaktion beeinflusst die elektrische Detektionsgröße. Die chemische Reaktion und damit die elektrische Detektionsgröße hängen davon ab, welche Konzentration der Bestandteil in der Gasprobe oder in der weiteren Gasprobe aufweist. Der elektrochemische Sensor vermag die elektrische Detektionsgröße zu messen. Der elektrochemische Sensor vermag ein Signal zu generieren, wobei das generierte Signal von der gemessenen elektrischen Detektionsgröße abhängt. Das generierte Signal umfasst eine Information über die Konzentration des Bestandteils in der Gasprobe oder in der weiteren Gasprobe. In einer Realisierungsform ist der gesuchte Bestandteil des Gasgemischs ein oxidierbarer Bestandteil, und die chemische Reaktion bewirkt, dass ein elektrischer Strom fließt. Die chemische Reaktion entspricht derjenigen chemischen Reaktion, die in einer Brennstoffzelle abläuft.

Die Ausgestaltung mit zwei Gassensoren, die unterschiedliche Messprinzipien anwenden, erbringt Redundanz. Falls ein Gassensor ausgefallen ist, lässt sich in vielen Fällen das Gasmessgerät mit dem anderen Gassensor dennoch verwenden. Außerdem lässt sich bei zwei Gassensoren eine Plausibilitätsprüfung durchführen. Wenn die beiden Sensoren signifikant unterschiedliche Messergebnisse für denselben Bestandteil der Gasprobe liefern, so ist dies ein Indiz dafür, dass mindestens ein Gassensor defekt ist oder defekt sein kann oder auch dafür, dass eine Fluidführungseinheit verstopft ist. Außerdem vermag ein Gasmessgerät mit zwei Sensoren, die unterschiedliche Messprinzipien anwenden, häufig mindestens zwei unterschiedliche Bestandteile in demselben Gasgemisch zuverlässig zu messen.

In einer bevorzugten Ausgestaltung vermag das erfindungsgemäße Gasmessgerät das Vorhandensein und / oder die Konzentration von Alkohol in dem eingegebenen Gasgemisch zu detektieren. Das Gasgemisch ist in dieser Anwendung eine Atemprobe eines Probanden. Der oder jeder Gassensor vermag bei dieser Ausgestaltung jeweils ein Maß für die Konzentration von Atemalkohol in der Atemprobe zu messen. Das Gasmessgerät ist also ein Alkoholmessgerät. In einer Ausgestaltung umfasst das Alkoholmessgerät die weiter oben beschriebenen zwei Sensoren, nämlich den photoelektrischen Sensor und den elektro-chemischen Sensor.

Bevorzugt ist das Gasmessgerät als ein tragbares Gerät ausgestaltet. Besonders bevorzugt lässt sich die Eingabeeinheit dergestalt vor den Mund eines Probanden halten, dass die Einlassöffnung zum Probanden hin zeigt. Bevorzugt umfasst das Gasmessgerät eine eigene Spannungsversorgungseinheit, um unabhängig von einem stationären Spannungsversorgungsnetz zu sein. Bevorzugt vermag das Gasmessgerät auf einer eigenen Ausgabeeinheit das Ergebnis der Messung auszugeben.

Im Folgenden wird die Erfindung anhand eines Ausführungsbeispiels beschrieben. Hierbei zeigt
- Figur 1: das Alkoholmessgerät in einer Seitenansicht von rechts;
- Figur 2: das Alkoholmessgerät von Figur 1, wobei der Grundkörper und die Fluidfördereinheit fortgelassen sind;
- Figur 3: das Alkoholmessgerät in einer Seitenansicht von links;
- Figur 4: das Alkoholmessgerät in einer Ansicht von links und schräg von unten;
- Figur 5: einen Ausschnitt aus einer Schnittdarstellung;
- Figur 6: das Alkoholmessgerät in einer Ansicht schräg hinten von links;
- Figur 7: das Mundstück und den Deckel von oben;
- Figur 8: den Deckel von oben bei abgenommenen Mundstück;
- Figur 9: einen Querschnitt durch das Gasmessgerät, wobei die Blickrichtung schräg von hinten ist;
- Figur 10: schematisch eine Fluidfördereinheit in Form einer Membranpumpe.

Im Ausführungsbeispiel wird die Erfindung eingesetzt, um zu prüfen, ob ein Proband Alkohol zu sich genommen hat. Bekanntlich enthält die Luft, die ein Mensch ausatmet, dann Alkohol, wenn der Proband zuvor Alkohol zu sich genommen hat und sich daher Alkohol in seinem Blut befindet. Die Erfindung wird beispielsweise dafür eingesetzt, um einen Fahrzeugführer oder Anlagenführer auf Atemalkohol zu untersuchen.

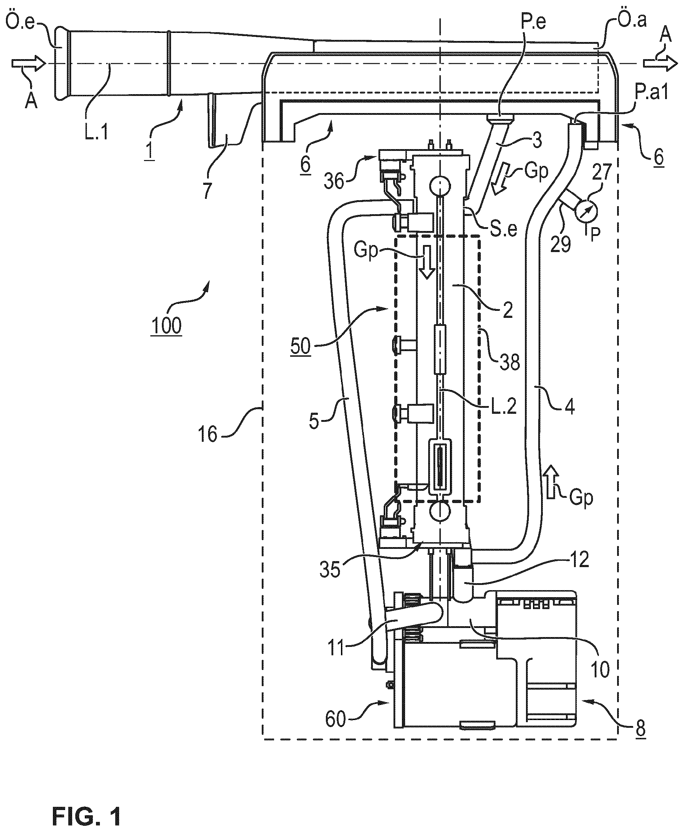

Figur 1 bis Figur 9 zeigen aus verschiedenen Betrachtungsrichtungen ein tragbares Alkoholmessgerät 100, in welchem die Erfindung verwendet wird, oder einen Detailausschnitt. Ein Mensch, beispielsweise ein Polizist oder der Proband selber, hält das Alkoholmessgerät 100 in einer Hand. Der Proband (nicht gezeigt) gibt eine Atemprobe A in ein röhrenförmiges Mundstück 1 ein. Das Mundstück 1 fungiert als die Eingabeeinheit, erstreckt sich entlang einer Längsachse L.1 und verjüngt sich weg vom Probanden. Die abgegebene Atemprobe A tritt durch eine Einlassöffnung Ö.e in das Mundstück 1 ein. Wenigstens ein Teil der Atemprobe A tritt durch eine Auslassöffnung Ö.a wieder aus dem Mundstück 1 heraus, wobei die Auslassöffnung Ö.a kleiner als die Einlassöffnung Ö.e ist. Die nachfolgend verwendeten Bezeichnungen "links", "rechts", "flussaufwärts" und "flussabwärts" beziehen sich auf die Fließrichtung der Atemprobe A durch das Mundstück 1 hindurch.

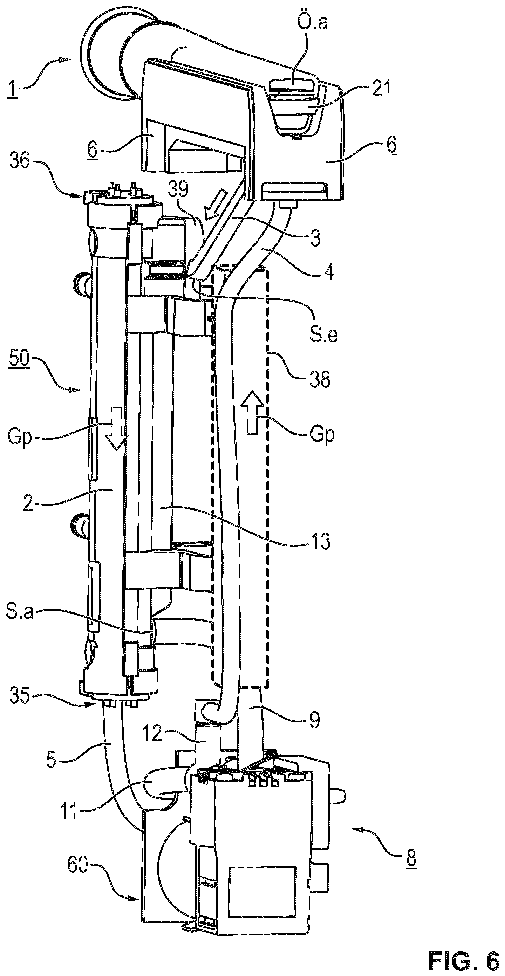

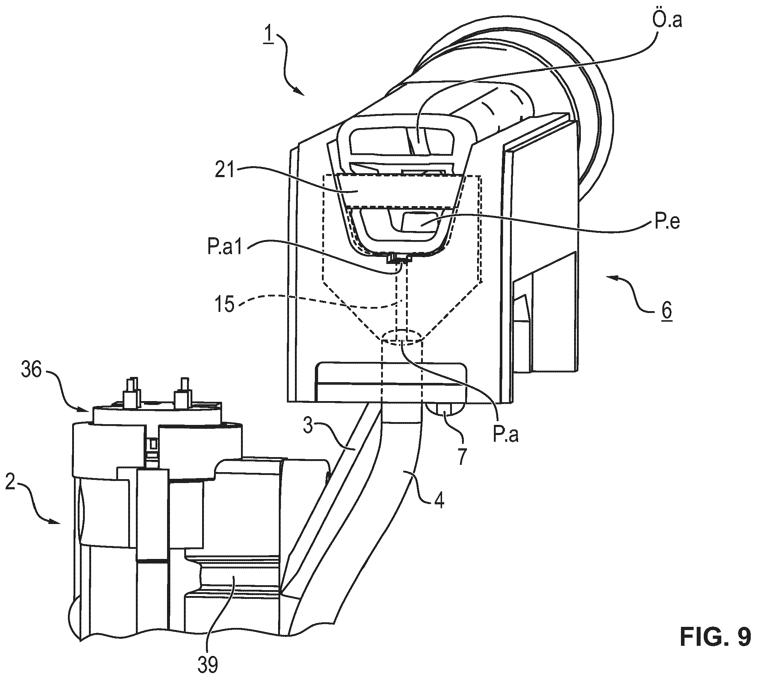

Im Ausführungsbeispiel ist die Einlassöffnung Ö.e kreisrund, während die Auslassöffnung Ö.a die Form eines Trapezes mit abgerundeten Ecken hat, vgl. Figur 6 und Figur 9.

Das Mundstück 1 ist lösbar mit einem annähernd quaderförmigen Grundkörper verbunden. Der Grundkörper umfasst ein zweiteiliges Gehäuse, von dem ein Deckel 6 perspektivisch und ein Basisteil 16 nur schematisch gezeigt werden. Das Mundstück 1 ist in den Deckel 6 eingerastet und lässt sich wieder vom Deckel 6 abnehmen. Ein Mensch hält das Basisteil 16 in einer Hand und hält die Einlassöffnung Ö.e vor den Mund des Probanden, damit der Proband die Atemprobe A in das Mundstück 1 eingeben kann. Wenigstens ein Teil der Atemprobe A tritt durch die Auslassöffnung Ö.a hindurch aus und gelangt wieder in die Umgebung. Möglich ist, dass der Rest der Atemprobe A am Mundstück 1 vorbeifließt und dann in die Umgebung austritt.

Das Mundstück 1 hat die Gestalt einer Röhre. Daher lassen sich das Basisteil 16 und damit auch das Mundstück 1 so positionieren und orientieren, dass die aus dem Mundstück 1 austretende Atemprobe A in eine gewünschte Richtung fließt. Dadurch ist die Gefahr relativ gering, dass die ausgetretene Atemprobe A dem Probanden oder einer anderen Person in der Nähe ins Gesicht fließt.

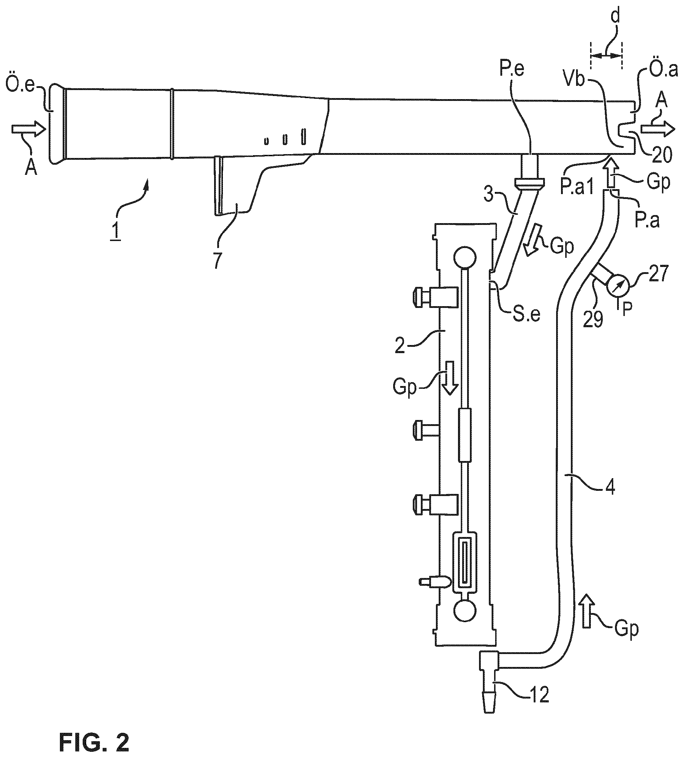

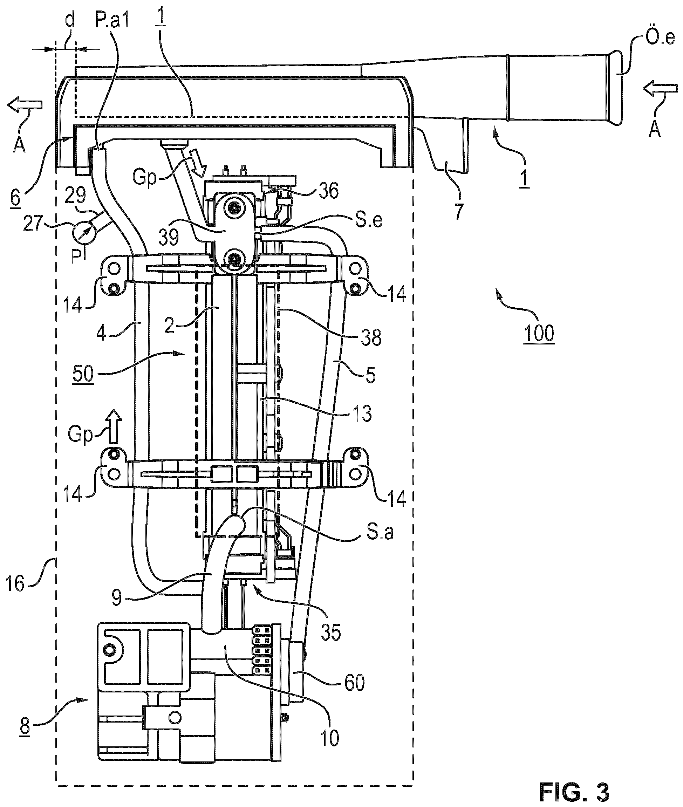

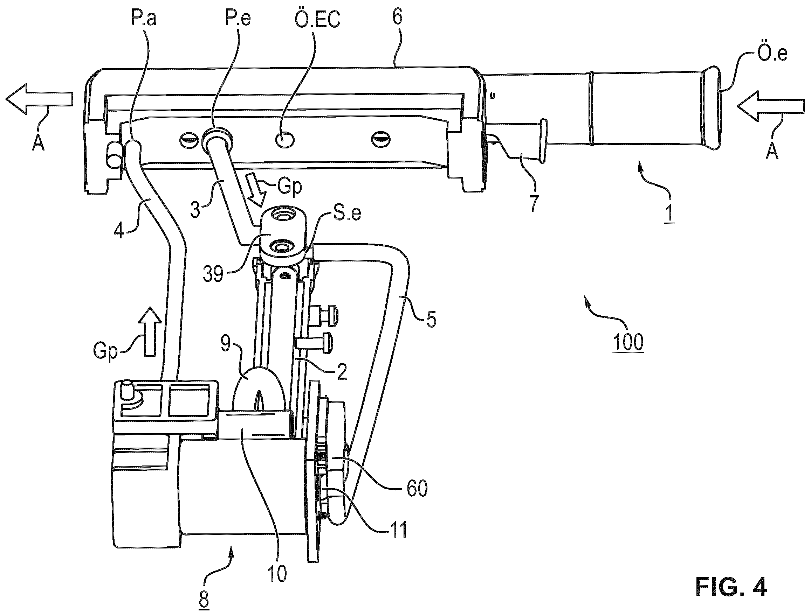

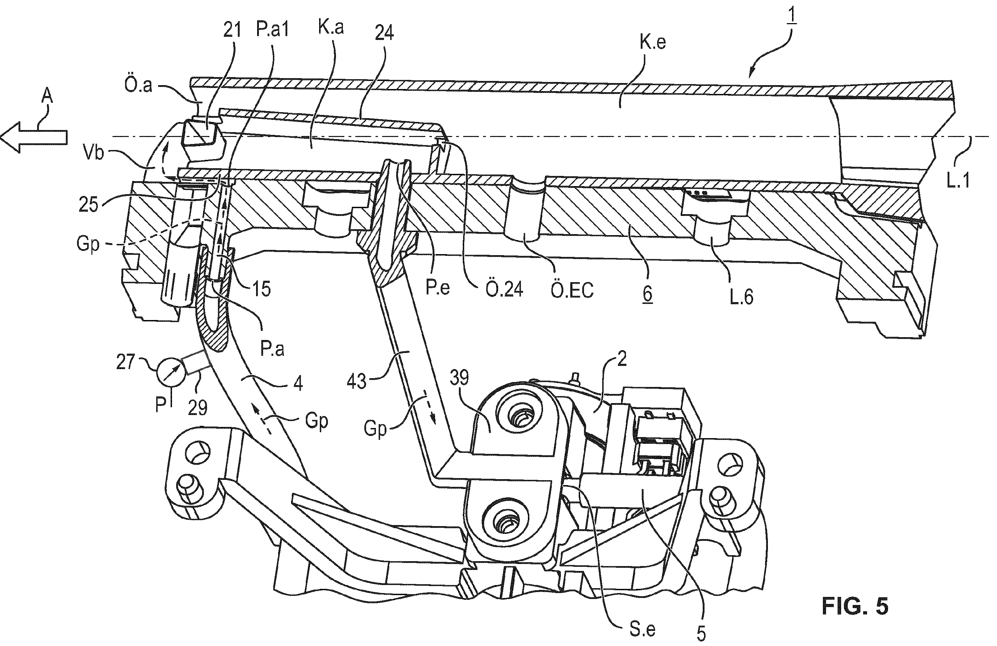

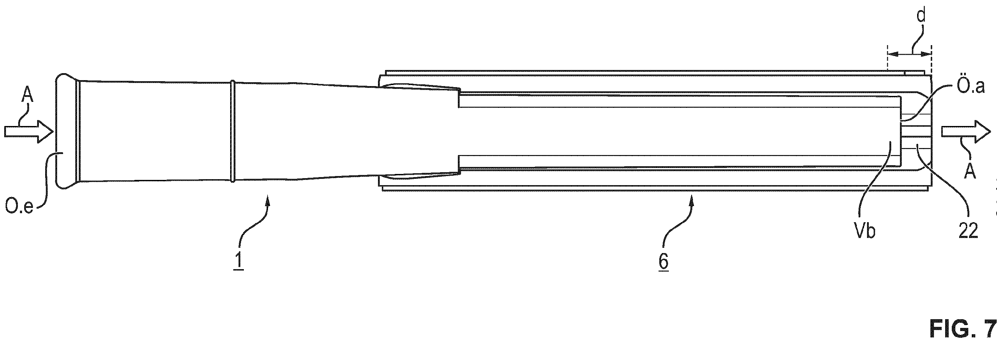

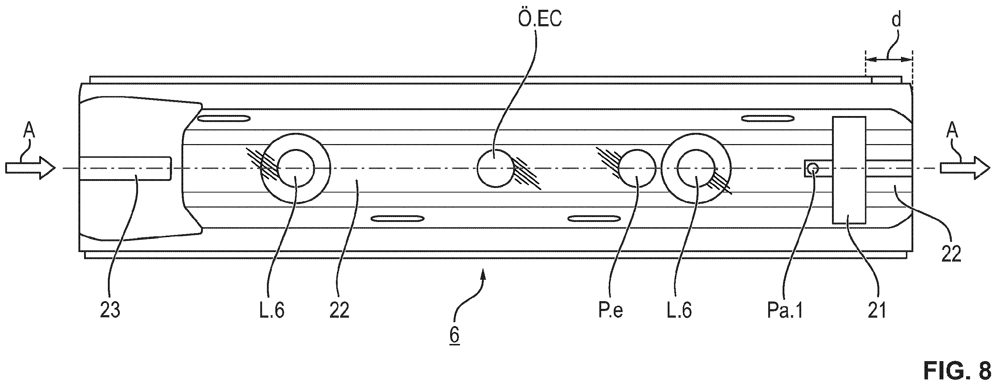

Figur 1 zeigt das Alkoholmessgerät 100 in einer Seitenansicht von rechts, d.h. die Atemprobe A fließt von links nach rechts durch das Mundstück 1. Figur 2 zeigt das Alkoholmessgerät 100 von Figur 1, wobei der Grundkörper 6, 16 fortgelassen ist. Figur 3 zeigt das Alkoholmessgerät 100 in einer Seitenansicht von links, d.h. die Atemprobe A fließt von rechts nach links durch das Mundstück 1. Figur 4 zeigt das Alkoholmessgerät 100 in einer Ansicht von links und von schräg unten, d.h. die Atemprobe A fließt von rechts nach links durch das Mundstück 1. Figur 5 zeigt einen Ausschnitt aus einer Schnittdarstellung, wobei die Blickrichtung von links ist. Figur 6 zeigt das Alkoholmessgerät 100 in einer Ansicht schräg von links, d.h. die Atemprobe A fließt schräg auf den Betrachter zu. Figur 7 zeigt von oben das Mundstück 1 und den Deckel 6, wobei die übrigen Bestandteile fortgelassen sind. Figur 8 zeigt von oben den Deckel 6, wobei zusätzlich das Mundstück 1 fortgelassen ist. In Figur 7 und Figur 8 ist zu sehen, dass der Deckel 6 flussabwärts vom Mundstück 1 um die Strecke d über das Mundstück 1 übersteht, und zwar an der Auslassöffnung Ö.a. Figur 9 zeigt in einer Betrachtungsrichtung schräg von hinten einen Querschnitt durch das Alkoholmessgerät 100, und zwar in Höhe einer weiter unten erläuterten Röhre 15.

Ein Trennelement 24 unterteilt das Mundstück 1 in eine einlassseitige Kammer K.e und eine auslassseitige Kammer K.a, vgl. Figur 5. Die einlassseitige Kammer K.e grenzt an die Einlassöffnung Ö.e an, die auslassseitige Kammer K.a an die Auslassöffnung Ö.a. Das Trennelement 24 trennt die beiden Kammern K.e, K.a fluiddicht voneinander, und zwar bis auf eine Öffnung Ö.24. Wenn der Proband eine Atemprobe A abgibt, tritt in der einlassseitigen Kammer K.e ein Staudruck auf. Ein nicht gezeigter Drucksensor misst ein Maß für diesen Staudruck. Der zeitliche Verlauf dieses Staudrucks zeigt an, ob und wenn ja wann und wie lange der Proband eine Atemprobe A in das Mundstück 1 eingibt. Dank des Trennelements 24 tritt in der auslassseitigen Kammer K.a ein Druck auf, der nur wenig vom Umgebungsdruck abweicht.

In der Realisierungsform, die in Figur 5 gezeigt wird, erstreckt sich die einlassseitige Kammer K.e von der Einlassöffnung Ö.e bis zur Auslassöffnung Ö.a. Die auslassseitige Kammer K.a ist in einer Realisierungsform durch eine Wand von der Auslassöffnung Ö.a getrennt. Gesehen in die Fließrichtung, in der die Atemprobe A durch das Mundstück 1 hindurchfließt, befindet die auslassseitige Kammer K.a sich neben demjenigen Segment der einlassseitigen Kammer K.e, das an die Auslassöffnung Ö.a angrenzt.

Ein Vorsprung 7 am Mundstück 1 greift in einen korrespondierenden Schlitz 23 im Deckel 6 ein, vgl. Figur 8. Im Mundstück 1 sind an der Auslassöffnung Ö.a zwei gegenüberliegende Schlitze 20 angeordnet. Ein Steg 21 des Deckels 6 greift bei eingesetztem Mundstück 1 in diese Schlitze 20 ein, vgl. Figur 6 und Figur 9.

Ein Teil der Atemprobe A gelangt aus der auslassseitigen Kammer K.a des Mundstücks 1 in eine Messküvette 2, welche als die Messkammer fungiert. Dieser Teil wird nachfolgend als die "Gasprobe Gp" bezeichnet. Die Messküvette 2 hat annähernd die Form eines Zylinders und erstreckt sich entlang einer Längsachse L.2, die im Ausführungsbeispiel senkrecht oder schräg auf der Längsachse L.1 des Mundstücks 1 steht. Wenn ein Mensch den Grundkörper 6, 16 in der Hand hält, ist die Längsachse L.2 annähernd vertikal angeordnet. Wie am besten in Figur 6 zu sehen ist, ist die Messküvette 2 relativ zum Mundstück 1 und zum Deckel 6 etwas seitlich nach rechts versetzt. Bevorzugt hat die Messküvette 2 eine Innenwand, welche elektromagnetische Strahlung reflektiert. Die Wand besteht aus einem Material, welches idealerweise nicht chemisch mit der Gasprobe reagiert, beispielsweise aus Gold oder einer Legierung, die Gold umfasst.

Die Messküvette 2 ist von einer Ummantelung 13 umgeben. Die Ummantelung 13 ist mit Hilfe von Befestigungselementen 14 an einer Innenwand des Grundkörpers 6, 16 befestigt. Die Ummantelung 13 ist in mehreren Figuren fortgelassen.

Mindestens ein Gassensor misst den Gehalt von Atemalkohol in der Gasprobe Gp, die sich in der Messküvette 2 befindet. Verschiedene Prinzipien, wie der Gassensor arbeiten kann, lassen sich anwenden, beispielsweise ein elektrochemischer Sensor, ein Infrarot-Sensor, ein ionisierender Sensor oder ein photoakustischer Sensor.

Im Ausführungsbeispiel umfasst das Alkoholmessgerät 100 einen optoelektrischen Sensor 50. Der Sensor 50 umfasst die Messküvette 2, eine Strahlungsquelle 35, einen Photodetektor 36 und optional einen Wellenlängenfilter (nicht gezeigt). Bevorzugt sind die Strahlungsquelle 35 und der Photodetektor 36 an zwei gegenüberliegenden Stirnwänden der Messküvette 2 angebracht, um einen großen optischen Weg zu erzielen. Im Ausführungsbeispiel sind die Strahlungsquelle 35 unten und der Photodetektor 36 oben angebracht.

Die Strahlungsquelle 35 emittiert elektromagnetische Strahlung, insbesondere Infrarotstrahlung, in die Messküvette 2 hinein. Die elektromagnetische Strahlung durchdringt mindestens einmal die Messküvette 2 und trifft auf den Photodetektor 36 auf. Der Photodetektor 36 erzeugt abhängig von der Intensität von auftreffender Strahlung ein analoges oder digitales Signal. Atemalkohol in der Messküvette 2 absorbiert in einem vorgegebenen Wellenlängenbereich einen Teil der elektromagnetischen Strahlung. Bevorzugt lässt der Wellenlängenfilter nur in diesem Wellenlängenbereich elektromagnetische Strahlung passieren. Dadurch wird die Gefahr reduziert, dass Wassertröpfchen in der Gasprobe Gp ein Messergebnis verfälschen.

Je größer die Konzentration von Atemalkohol ist, desto mehr elektromagnetische Strahlung wird im betreffenden Wellenlängenbereich in der Messküvette 2 absorbiert. Daher ist das Signal des Photodetektors 36 ein Maß für die Konzentration von Atemalkohol in der Gasprobe Gp. Eine Auswerteeinheit eines signalverarbeitenden Steuergeräts (control unit) 60 wertet das Signal des Photodetektors 36 aus und erzeugt ein Signal, welches eine Information über den Gehalt von Atemalkohol in der Gasprobe Gp enthält. Bevorzugt gibt eine nicht gezeigte Ausgabeeinheit des Alkoholmessgeräts 100 diese Informationen in mindestens einer von einem Menschen wahrnehmbaren Form aus.

Die Gasprobe Gp wird in einem Eintrittspunkt P.e von der Atemprobe A, welche durch das Mundstück 1 fließt, abgezweigt. Bevorzugt werden höchstens 10 Vol.-% der Atemprobe A als Gasprobe abgezweigt, besonders bevorzugt höchstens 2 Vol.-%. Die Gasprobe Gp fließt durch eine Zuführleitung 3 und ein Verbindungsstück 39 hindurch in die Messküvette 2, durch die Messküvette 2 hindurch und durch eine Abführleitung 4 hindurch wieder aus der Messküvette 2 heraus. Im Ausführungsbeispiel ist die Zuführleitung 3 eine starre Röhre und wird beheizt, beispielsweise durch Heizelemente in der Wand der Röhre 3 und / oder im Verbindungsstück 39. Die Abführleitung 4 ist ein flexibler Schlauch.

Figur 1 bis Figur 6 veranschaulichen mithilfe von Pfeilen den Weg der Gasprobe Gp. Die Gasprobe Gp tritt im Eintrittspunkt P.e in die Zuführleitung 3 ein und fließt durch die Zuführleitung 3, durch die Messküvette 2 und die Abführleitung 4 hindurch bis zu einem Austrittspunkt P.a, vgl. Figur 2 und Figur 4. Anschließend fließt die Gasprobe durch eine Röhre 15 im Deckel 6 bis zu einem Austrittspunkt P.a1. In einer ersten Alternative, die nicht vom Schutzumfang umfasst wird, tritt die Gasprobe Gp im Austrittspunkt P.a1 wieder in das Mundstück 1 ein, vgl. Figur 2 und Figur 9. Figur 9 zeigt einen Querschnitt durch das Alkoholmessgerät 100 in Höhe des Austrittspunkts P.a1, wobei die Längsachse L.1 des Mundstücks 1 fast senkrecht auf der Zeichenebene steht.

In einer zweiten, erfindungsgemäßen Alternative fließt die Gasprobe Gp nicht wieder in das Mundstück 1 hinein. Vielmehr fließt die Gasprobe Gp vom Austrittspunkt P.a1 in einem Schlitz 25 zwischen dem Mundstück 1 und dem Deckel 6 in Richtung der Auslassöffnung Ö.a. Die Gasprobe Gp fließt also am Mundstück 1 vorbei in eine Umgebung des Alkoholmessgeräts 100.

In beiden Alternativen ist der Austrittspunkt P.a1 mit einem Abstand flussabwärts vom Eintrittspunkt P.e angeordnet.

Im Ausführungsbeispiel umfasst das Alkoholmessgerät 100 zusätzlich zum gerade beschriebenen optoelektrischen Sensor 50 mit der Messküvette 2, der Strahlungsquelle 35 und dem Photodetektor 36 einen elektrochemischen Sensor 38, der nur schematisch gezeigt wird. Eine weitere Gasprobe tritt durch eine Öffnung Ö.EC im Deckel 6 aus dem Mundstück 1 aus und erreicht eine nicht gezeigte Messkammer des elektrochemischen Sensors 38. In dieser Messkammer findet eine chemische Reaktion statt, die eine elektrische Detektionsgröße beeinflusst. Bevorzugt ist die chemische Reaktion diejenige, die auch in einer Brennstoffzelle stattfindet. Diese elektrische Detektionsgröße wird gemessen und ist ein Maß für die Konzentration von Atemalkohol in der weiteren Gasprobe.

Das Merkmal, dass das Alkoholmessgerät 100 sowohl einen optoelektrischen Sensor 50 als auch einen elektrochemischen Sensor 38 umfasst, hat insbesondere folgende Vorteile:
- Die beiden Sensoren stellen Redundanz her. Falls ein Gassensor 50 oder 38 ausfällt, kann der andere Gassensor 38 oder 50 immer noch den Gehalt von Atemalkohol in der Atemprobe A messen.
- Die Messwerte der beiden Sensoren 50, 38 lassen sich miteinander vergleichen. Falls die Messwerte sehr stark voneinander abweichen, so kann dies ein Indiz dafür sein, dass mindestens ein Gassensor 50, 38 defekt ist.
- Möglich ist, dass in der Atemprobe A und damit in der Gasprobe Gp außer Atemalkohol ein weiterer Bestandteil enthalten ist, auf den die beiden Gassensoren 50 und 38 unterschiedlich reagieren. In vielen Fällen lässt sich dieser weitere Bestandteil zuverlässig von Atemalkohol unterscheiden, wenn zwei verschiedene Sensoren 50 und 38 verwendet werden.
- Durch die Messküvette 2 fließt ein Strom von Gas, so dass sich zu jedem Zeitpunkt in der Messküvette 2 eine andere Gasprobe Gp befindet. Der optoelektrische Sensor 50 misst kontinuierlich, genauer gesagt: mit einer festen Abtastfrequenz, den Gehalt von Atemalkohol. Das Signal des optoelektrischen Sensors 50 liefert daher einen zeitlichen Verlauf des Atemalkohols in der Gasprobe Gp. Dies ermöglicht es in vielen Fällen, den Gehalt von Atemalkohol in verschiedenen Teilen der Atemprobe A voneinander zu unterscheiden. Die Atemprobe A umfasst einen Teil, der aus dem Mund des Probanden stammt, anschließend einen Teil, der aus den oberen Atemwegen stammt, und abschließend einen Teil, der aus der Lunge stammt. Oft wird die Information gewünscht, ob sich ein Messwert für den Alkoholgehalt auf eine Atemprobe aus dem Mund, den oberen Atemwegen oder der Lunge bezieht. Der elektrochemische Sensor 38 vermag aufgrund seines Messprinzips den Gehalt an Atemalkohol nur mit einer geringeren Abtastfrequenz zu messen als der optoelektrische Sensor 50.

Die nachfolgende Beschreibung bezieht sich wiederum auf die Gasprobe Gp, die vom optoelektrischen Sensor 50 untersucht wird. Das Entsprechende gilt für die andere Gasprobe, die vom optionalen elektrochemischen Sensor 38 untersucht wird. Die Gasprobe Gp vermischt sich in einem Vermischungsbereich Vb mit demjenigen Teil der Atemprobe A, der von der Einlassöffnung Ö.e durch das Mundstück 1 hindurch zur Auslassöffnung Ö.a geflossen ist, ohne abgezweigt zu werden. Bei der ersten Alternative erstreckt sich dieser Vermischungsbereich Vb im Mundstück 1 vom Austrittspunkt P.a1 bis zur Auslassöffnung Ö.a, vgl. Figur 2 und Figur 9. Bei der zweiten Alternative ist der Vermischungsbereich Vb flussabwärts vom Mundstück 1 und oberhalb der Rinne 22 angeordnet, vgl. Figur 5 und Figur 7.

Die zweite Alternative hat insbesondere folgenden Vorteil: Der Vermischungsbereich Vb, in dem sich die Gasprobe Gp mit dem Rest der Atemprobe A vermischt, liegt bei der zweiten Alternative flussabwärts vom Mundstück 1 und daher außerhalb des Mundstücks 1. Daher ist die Gefahr verringert, dass in der auslassseitigen Kammer K.a des Mundstücks 1 Verwirbelungen und / oder ein Rückstau auftreten.

In den Deckel 6 sind mehrere Öffnungen eingelassen, darunter
- eine Öffnung für den Eintrittspunkt P.e, in dem die Zuführleitung 3 zum optoelektrischen Sensor 50 beginnt,
- eine Öffnung Ö.EC, in der eine nicht gezeigte Zuführleitung zum elektrochemischen Sensor 38 beginnt, und
- bei der ersten Alternative eine Öffnung für den Austrittspunkt P.a1, in den die Röhre 15 mündet, vgl. Figur 8 und Figur 9.

Außerdem sind in den Deckel 6 mehrere Löcher L.6 für eine Schraubverbindung mit dem Basisteil 16 eingelassen, vgl. Figur 5 und Figur 8.

In den Deckel 6 ist eine Rinne 22 eingelassen, vgl. Figur 7 und Figur 8. Diese Rinne 22 erstreckt sich entlang der gesamten Längsachse des Deckels 6. Falls das Mundstück 1 auf den Deckel 6 aufgesetzt ist, greift das Mundstück 1 in die Rinne 22 ein und verschließt mehrere Öffnungen im Deckel 6, vgl. Figur 5. Die beiden Öffnungen für den Eintrittspunkt P.e und den Austrittspunkt P.a1 (nur erste Alternative) sowie die Öffnung Ö.EC werden nicht verschlossen.

Möglich ist, dass der Überdruck, den der Proband durch das Ausatmen im Mundstück 1 erzeugt, ausreichend ist, um die Gasprobe in die Messküvette 2 zu leiten. Im Ausführungsbeispiel wird hingegen bevorzugt eine Fluidfördereinheit 8 verwendet, um die Gasprobe Gp aus der Atemprobe A, die durch das Mundstück 1 fließt, abzuzweigen, in die Messküvette 2 zu fördern und wieder aus der Messküvette 2 auszustoßen. Die Fluidfördereinheit 8 saugt Gas durch eine Leitung 9 aus der Messküvette 2. Dadurch wird ein Unterdruck erzeugt, und die Gasprobe Gp fließt durch die Zuführleitung 3 in die Messküvette 2. In vielen Fällen füllt die Gasprobe Gp die Messküvette 2 relativ gleichmäßig aus, was die Zuverlässigkeit der Messung erhöht. Die angesaugte Gasprobe Gp fließt aus der Messküvette 2 durch einen Schlauch 9 und eine starre Leitung 10 in eine Kammer der Fluidfördereinheit 8. Anschließend fließt die Gasprobe Gp aus der Kammer der Fluidfördereinheit 8 durch eine Röhre 12 in die Abführleitung 4.

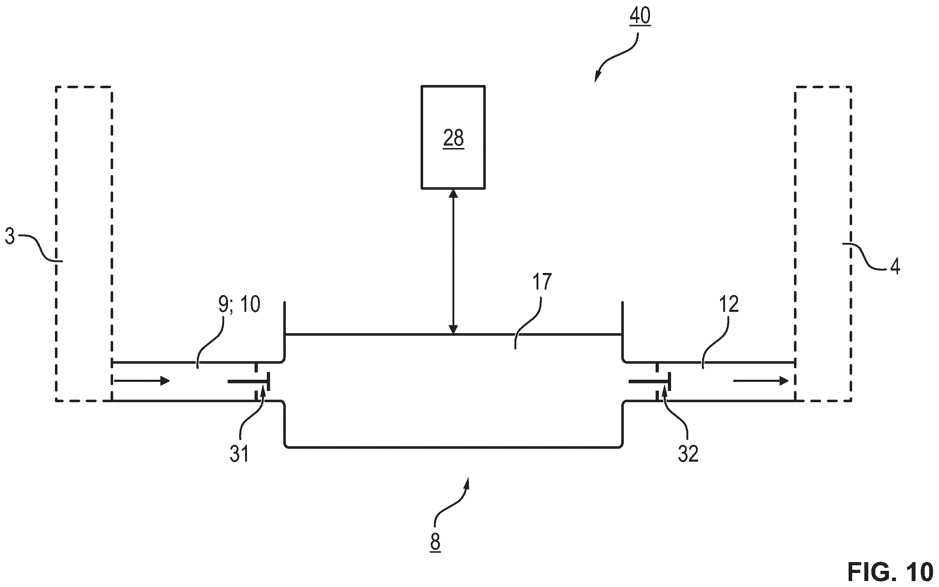

Verschiedene Ausgestaltungen der Fluidfördereinheit 8 sind möglich. Im Ausführungsbeispiel umfasst die Fluidfördereinheit 8 eine Membranpumpe 40, die schematisch in Figur 10 dargestellt wird. Die Membranpumpe 40 umfasst eine Kammer 17 mit einem veränderbaren Volumen sowie einen schematisch gezeigten Stellantrieb 28, welcher das Volumen der Kammer 17 zu verändern vermag. Die Zuführung 9, 10 mündet in dieser schematischen Darstellung von links in die Kammer 17, und die Abführung 12 führt rechts aus der Kammer 17 heraus. Ein Ansaug-Rückschlagventil 31 gibt einen Fluss von Gas in die Kammer 17 frei und versperrt einen Fluss in die entgegengesetzte Richtung. Entsprechend gibt ein Ausstoß-Rückschlagventil 32 einen Fluss von Gas aus der Kammer 17 heraus frei und versperrt einen Fluss in die entgegengesetzte Richtung.

Im Ausführungsbeispiel wird der Volumenfluss der Gasprobe Gp durch die Messküvette 2 hindurch gemessen. Durch Aufintegrieren über die Zeit leitet das Steuergerät 60 aus dem gemessenen Volumenfluss näherungsweise das Volumen her, das seit einem bestimmten Ereignis in die Messküvette 2 geflossen ist, beispielsweise seit dem Aufsetzen des Mundstücks 1. Ein Messergebnis des Alkoholmessgeräts 100 ist nur dann gültig, wenn eine Gasprobe Gp mit einem ausreichend großen Volumen in die Messküvette 2 gelangt ist.

Unter dem "Volumenfluss", auch Volumenstrom genannt, wird das Volumen pro Zeiteinheit des Fluids verstanden, welches durch eine Fluidführungseinheit, beispielsweise durch die Messküvette 2, hindurchfließt. Um den Volumenfluss zu messen, wird mit einer bestimmten Abtastfrequenz eine zeitlich veränderliche Druckdifferenz gemessen, und zwar eine Differenz zwischen dem Druck an der Stelle S.e, an dem das Fluid aus der Zuführleitung 3 austritt und in die Messküvette 2 eintritt, und dem Druck an der Stelle S.a, an der das Fluid aus der Messküvette 2 austritt und in die Leitung 9 eintritt, vgl. Figur 3. Diese Druckdifferenz ist ein Maß für den Volumenfluss. Nachfolgend wird eine Realisierungsform beschrieben, um diese Druckdifferenz zu messen.

Eine Messleitung 5 steht in einer Fluidverbindung mit der Austrittsöffnung der Zuführleitung 3. Daher herrscht in der Messleitung 5 der gleiche Druck wie an der Stelle S.e, an der das Fluid in die Messküvette 2 eintritt. In der Leitung 9 herrscht der gleiche Druck wie an der Stelle S.a, an der das Fluid aus der Messküvette 2 austritt. Ein Sensor für die Druckdifferenz steht in einer Fluidverbindung mit den Leitungen 9 und 5. Messwerte betreffend die Druckdifferenz werden an das Steuergerät 60 übermittelt, und das Steuergerät 60 leitet aus diesen Messwerten den Volumenfluss her und erzeugt ein Signal, das den gemessenen Volumenfluss umfasst. Eine Leitung 11 verbindet die Messleitung 5 pneumatisch mit der Leitung 10.

Nachdem die Atemprobe A eines Probanden untersucht worden ist, ist es erforderlich, die Messküvette 2 auszuspülen. Im Ausführungsbeispiel wird die Fluidfördereinheit 8 auch dafür verwendet, um die Messküvette 2 auszuspülen. Beim Ausspülen fließt Umgebungsluft auf dem gleichen Weg durch die Messküvette 2 wie eine zu untersuchende Gasprobe Gp. Die Messküvette 2 lässt sich mit verbundenem Mundstück 1 oder ohne Mundstück 1 spülen. Bevorzugt wird auch beim Ausspülen der Volumenfluss gemessen, um sicherzustellen, dass beim Ausspülen eine ausreichende Menge von Umgebungsluft durch die Messküvette 2 fließt.

Möglich ist auch, dass die Fluidfördereinheit 8 ausschließlich dafür verwendet wird, um die Messküvette 2 auszuspülen. Wie bereits dargelegt, reicht in einer Ausgestaltung der Druck, mit dem ein Proband die Atemprobe A abgibt, aus, damit die zu untersuchende Gasprobe Gp in die Messküvette 2 gelangt. Die Ausgestaltung, eine Fluidfördereinheit 8 ausschließlich zum Spülen der Messküvette 2 zu verwenden, macht es möglich, eine Fluidfördereinheit zu verwenden, die weniger Bauraum einnimmt und weniger elektrische Energie verbraucht, verglichen mit einer Fluidfördereinheit, welche eine Atemprobe A ansaugt.

Der Gehalt an Atemalkohol kann nur dann korrekt gemessen werden, wenn das Mundstück 1 korrekt auf den Deckel 6 aufgesetzt ist und einrastet. Das Mundstück 1 greift dann in die Rinne 22 ein. Bevorzugt sollte die Fluidfördereinheit 8 sowohl dann eingeschaltet sein, wenn die Gasprobe Gp aus der Atemprobe A abgezweigt wird, als auch dann, wenn die Messküvette 2 gespült wird. Die nachfolgend beschriebene Ausgestaltung ermöglicht es, automatisch zu überprüfen, ob diese Voraussetzungen aktuell erfüllt sind oder nicht.

Vorgegeben werden ein kleinerer Druck-Grenzwert x und ein größerer Druck-Grenzwert y. Ein Drucksensor 27 vermag ein Maß für den Druck in der Abführleitung 4 zu messen. Dieser Drucksensor 27 kann zu dem oben beschriebenen Sensor für die Druckdifferenz gehören, wobei die Druckdifferenz gemessen wird, um den Volumenfluss in die und aus der Messküvette 2 zu messen. Der Drucksensor 27 ist über eine Verbindungsleitung 29 pneumatisch mit der Abführleitung 4 verbunden.

Das Steuergerät 60 vergleicht den gemessenen Druck mit den beiden Druck-Grenzwerten x und y. Falls der gemessene Druck kleiner ist als der kleinere Druck-Grenzwert x, so ist die Fluidfördereinheit 8 ausgeschaltet. Falls der gemessene Druck zwischen den beiden Druck-Grenzwerten x und y liegt, so ist die Fluidfördereinheit 8 eingeschaltet, und kein Mundstück 1 ist auf den Deckel 6 aufgesetzt. Oder das Mundstück 1 ist nicht richtig aufgesetzt, beispielsweise nicht eingerastet, so dass ein zu großer Abstand zwischen dem Mundstück 1 und dem Deckel 6 auftritt. Falls der gemessene Druck größer ist als der größere Druck-Grenzwert y, so ist die Fluidfördereinheit 8 eingeschaltet, und ein Mundstück 1 ist aufgesetzt. Der Grund für diese Unterscheidung: Bei aufgesetztem Mundstück 1 tritt ein größerer Druck in der Abführleitung 4 auf als bei einem fehlenden Mundstück 1. Die Druck-Grenzwerte x und y werden bevorzugt vorab empirisch ermittelt.

Bevorzugt gibt das Alkoholmessgerät 100 eine Meldung in einer von einem Menschen wahrnehmbaren Form aus, falls das Alkoholmessgerät 100 eine Atemprobe A aufnehmen und untersuchen soll, jedoch ein Mundstück 1 gar nicht oder nicht korrekt aufgesetzt ist.

### Bezugszeichenliste

| | |
|---|---|
| 1 | röhrenförmiges Mundstück, das sich lösbar auf den Deckel 6 aufsetzen lässt, umfasst den Vorsprung 7 und den Steg 21, erstreckt sich entlang der Längsachse L.1, lösbar mit dem Deckel 6 verbunden, fungiert als die Eingabeeinheit |
| 2 | Messküvette, gehört zum optoelektrischen Sensor 50, erstreckt sich entlang der Längsachse L.2, fungiert als die Messkammer |
| 3 | Zuführleitung, gehört zur Zuführ-Fluidführungseinheit |
| 4 | Abführleitung, gehört zur Abführ-Fluidführungseinheit |
| 5 | Messleitung |
| 6 | Deckel des Alkoholmessgeräts 100, trägt das Mundstück 1, bildet zusammen mit dem Basisteil 16 den Grundkörper des Alkoholmessgeräts 100, weist die Löcher L.6 auf |
| 7 | Vorsprung am Mundstück 1, greift in den Schlitz 23 ein |
| 8 | Fluidfördereinheit, saugt die Gasprobe Gp aus dem Mundstück 1 ab, in einer Ausführungsform als Membranpumpe 40 ausgestaltet |
| 9 | Leitung von der Leitung 10 zur Messküvette 2 |
| 10 | Leitung von der Kammer 17 zur Leitung 9 |
| 11 | Leitung, verbindet die Messleitung 5 mit der Leitung 10 |
| 12 | Röhre, führt von der Kammer 17 zur Abführleitung 4 |
| 13 | Ummantelung der Messküvette 2 |
| 14 | Befestigungselemente für die Ummantelung 13 |
| 15 | Röhre im Deckel 6 |
| 16 | Basisteil, bildet zusammen mit dem Deckel 6 den Grundkörper des Alkoholmessgeräts 100 |
| 17 | Kammer der Membranpumpe 40 |
| 20 | Schlitze im Mundstück 1 an der Auslassöffnung Ö.a |
| 21 | Steg des Deckels 6, greift in die Schlitze 20 ein |
| 22 | Rinne im Deckel 6 |
| 23 | Schlitz im Deckel 6, in den der Vorsprung 7 eingreift |
| 24 | Trennelement, welches das Mundstück 1 in eine einlassseitige Kammer K.e und eine auslassseitige Kammer K.a unterteilt, weist die Öffnung Ö.24 auf |
| 25 | Schlitz zwischen dem Mundstück 1 und dem Deckel 6, fungiert als Kanal |
| 27 | Drucksensor, misst den Druck in der Abführleitung 4 |
| 28 | Stellantrieb der Membranpumpe 40 |
| 29 | Verbindungsleitung zwischen dem Drucksensor 27 und der Abführleitung 4 |
| 31 | Ansaug-Rückschlagventil der Membranpumpe 40 |
| 32 | Ausstoß-Rückschlagventil der Membranpumpe 40 |
| 35 | Strahlungsquelle des optoelektrischen Sensors 50 |
| 36 | Photodetektor des optoelektrischen Sensors 50 |
| 38 | elektrochemischer Sensor |
| 39 | Verbindungsstück zwischen der Zuführleitung 3 und der Messküvette 2 |
| 40 | Membranpumpe, umfasst die Kammer 17, den Stellantrieb 28, das Ansaug-Rückschlagventil 31 und das Ausstoß-Rückschlagventil 32 |
| 50 | optoelektrischer Sensor, umfasst die Messküvette 2, die Strahlungsquelle 35 und den Photodetektor 36 |
| 60 | Steuergerät des Alkoholmessgeräts 100 |
| 100 | Alkoholmessgerät, umfasst das Mundstück 1, das Basisteil 16, den Deckel 6, die Sensoren 50 und 38, die Fluidfördereinheit 8, die Leitungen 3, 4, 5, 9, 10, 11 und das Steuergerät 60 |
| A | Atemprobe, tritt durch die Einlassöffnung Ö.e in das Mundstück ein und verlässt durch die Auslassöffnung Ö.a des Mundstücks 1 |
| d | Strecke, über die der Deckel 6 über das Mundstück 1 übersteht |
| Gp | Gasprobe, fließt durch die Zuführleitung 3 in die Messküvette 2, durch die Messküvette 2 und durch die Abführleitung 4 |
| K.a | auslassseitige Kammer im Mundstück 1 |
| K.e | einlassseitige Kammer im Mundstück 1 |
| L.1 | Längsachse des Mundstücks 1 |
| L.2 | Längsachse der Messküvette 2 |
| L.6 | Löcher im Deckel 6 für eine Schraubverbindung mit dem Basisteil 16 |
| Ö.e | Einlassöffnung des Mundstücks 1 |
| Ö.a | Auslassöffnung des Mundstücks 1 |
| Ö.24 | Öffnung im Trennelement 24 |
| Ö.EC | Öffnung im Deckel 6, in der eine Zuführleitung zum elektrochemischen Sensor 38 beginnt |
| P.a | Austrittspunkt aus der Abführleitung 4 |
| P.e | Eintrittspunkt in die Zuführleitung 3 |
| P.a1 | Austrittspunkt aus der Röhre 15 |
| S.a | Stelle ,an der das Fluid aus der Messküvette 2 austritt |
| S.e | Stelle, an der das Fluid in die Messküvette 2 eintritt |
| Vb | Vermischungsbereich, in dem die abgezweigte Gasprobe Gp sich mit dem Rest der Atemprobe A vermischt |
| x | kleinerer Druck-Grenzwert |
| y | größerer Druck-Grenzwert |

## Patentansprüche

1. Gasmessgerät (100) zum Messen der Konzentration mindestens eines Bestandteils in einem Gasgemisch (A),
wobei das Gasmessgerät (100)
- eine röhrenförmige Eingabeeinheit (1),
- einen Grundkörper (6, 16),
- eine Zuführ-Fluidführungseinheit (3, 39),
- eine Messkammer (2),
- eine Abführ-Fluidführungseinheit (4, 15) und
- mindestens einen Gassensor (50, 38)
umfasst,
wobei die Eingabeeinheit (1)
- eine Längsachse (L.1), eine Einlassöffnung (Ö.e) und eine Auslassöffnung (Ö.a) aufweist und
- mit dem Grundkörper (6, 16) verbunden oder verbindbar ist,
wobei die Messkammer (2) im Inneren des Grundkörpers (6, 16) angeordnet ist,
wobei die Zuführ-Fluidführungseinheit (3, 39) einen Eintrittspunkt (P.e) im Grundkörper (6, 16) mit der Messkammer (2) verbindet und die Abführ-Fluidführungseinheit (4, 15) die Messkammer (2) mit einem Austrittspunkt (P.a1) im Grundkörper (6, 16) verbindet,
wobei die beiden Fluidführungseinheiten (3, 39; 4, 15) voneinander beabstandet sind,
wobei das Gasmessgerät (100) dergestalt ausgestaltet ist, dass
- ein Gasgemisch (A) durch die Einlassöffnung (Ö.e) in die Eingabeeinheit (1) hinein und durch die Eingabeeinheit (1) hindurch fließt,
- eine Gasprobe (Gp) im Eintrittspunkt (P.e) aus dem durchfließenden Gasgemisch (A) abgezweigt wird,
- der übrige Teil des Gasgemischs (A), also das Gasgemisch (A) ohne die abgezweigte Gasprobe (Gp), am Eintrittspunkt (P.e) vorbei fließt,
wobei das Gasmessgerät (100) weiterhin dergestalt ausgestaltet ist, dass die abgezweigte Gasprobe (Gp)
- vom Eintrittspunkt (P.e) durch die Zuführ-Fluidführungseinheit (3, 39) hindurch in die Messkammer (2) und
- aus der Messkammer (2) durch die Abführ-Fluidführungseinheit (4, 15) hindurch zum Austrittspunkt (P.a1) fließt, und
wobei das Gasmessgerät (100) weiterhin dergestalt ausgestaltet ist, dass
- die abgezweigte Gasprobe (Gp) im Austrittspunkt (P.a1) aus dem Grundkörper (6, 16) heraus und an der Eingabeeinheit (1) vorbei fließt und
- sowohl die abgezweigte Gasprobe (Gp) als auch der übrige Teil des Gasgemischs (A) an zwei voneinander beabstandeten Stellen in eine Umgebung des Gasmessgeräts (100) fließen, und
wobei der oder jeder Gassensor (50, 38) dazu ausgestaltet ist, jeweils die Konzentration des Bestandteils in der Gasprobe (Gp) in der Messkammer (2) zu messen,
**dadurch gekennzeichnet, dass**
das Gasmessgerät (100) so ausgestaltet ist, dass dann, wenn die Eingabeeinheit (1) mit dem Grundkörper (6, 16) verbunden ist, ein Kanal (25) zwischen dem Grundkörper (6, 16) und der Eingabeeinheit (1) auftritt,
wobei dieser Kanal (25) den Austrittspunkt (P.a1) mit einer Umgebung des Gasmessgeräts (100) verbindet und sich parallel zur Längsachse (L.1) der Eingabeeinheit (1) erstreckt und
wobei das Gasmessgerät (100) weiterhin dergestalt ausgestaltet ist, dass
die abgezweigte Gasprobe (Gp) beim Fließen an der Eingabeeinheit (1) vorbei in eine Richtung weg von der Einlassöffnung (Ö.e) und dabei vom Austrittspunkt (P.a1) durch den Kanal (25) hindurch fließt.

2. Gasmessgerät (100) nach Anspruch 1,
**dadurch gekennzeichnet, dass**
der Grundkörper (6, 16) - gesehen in eine Fließrichtung des Gasgemischs (A) durch die Eingabeeinheit (1) hindurch - flussabwärts von der Eingabeeinheit (1) über die Eingabeeinheit (1) übersteht,
so dass flussabwärts von der Auslassöffnung (Ö.a) ein Bereich (Vb) gebildet wird, der auf einer Seite von der Eingabeeinheit (1) und auf einer anderen Seite vom Grundkörper (6, 16) begrenzt ist.

3. Gasmessgerät (100) nach einem der vorhergehenden Ansprüche,
**dadurch gekennzeichnet, dass**
ein Trennelement (24) die Eingabeeinheit (1) in eine einlassseitige Kammer (K.e) und eine auslassseitige Kammer (K.a) unterteilt,
wobei die Einlassöffnung (Ö.e) an die einlassseitige Kammer (K.e) angrenzt,
wobei die Auslassöffnung (Ö.a) an die auslassseitige Kammer (K.a) angrenzt,
wobei mindestens eine Durchlassöffnung (Ö.24) im Trennelement (24) die beiden Kammern (K.e, K.a) miteinander verbindet und
wobei sowohl der Eintrittspunkt (P.e) als auch der Austrittspunkt (P.a1) an die auslassseitige Kammer (K.a) angrenzen.

4. Gasmessgerät (100) nach Anspruch 3,
**dadurch gekennzeichnet, dass**
die einlassseitige Kammer (K.e) die Einlassöffnung (Ö.e) mit der Auslassöffnung (Ö.a) verbindet und
gesehen in eine Fließrichtung des Gasgemischs (A) durch die Eingabeeinheit (1) hindurch sich ein Segment der einlassseitigen Kammer (K.e) neben der auslassseitigen Kammer (K.a) befindet.

5. Gasmessgerät (100) nach einem der vorhergehenden Ansprüche,
**dadurch gekennzeichnet, dass**
die Eingabeeinheit (1) lösbar mit dem Grundkörper (6, 16) verbunden oder verbindbar ist und
das Gasmessgerät (100) einen Drucksensor (27) umfasst,
wobei der Drucksensor (27) dazu ausgestaltet ist, den Druck in der Zuführ-Fluidführungseinheit (3, 39) oder den Druck in der Abführ-Fluidführungseinheit (4, 15) zu messen, und
wobei das Gasmessgerät (100) dazu ausgestaltet ist, abhängig vom gemessenen Druck automatisch zu entscheiden, ob die Eingabeeinheit (1) mit dem Grundkörper (6, 16) verbunden ist oder nicht.

6. Gasmessgerät (100) nach einem der vorhergehenden Ansprüche,
**dadurch gekennzeichnet, dass**
das Gasmessgerät (100) einen Drucksensor (27) und eine Fluidfördereinheit (8) umfasst,
wobei die Fluidfördereinheit (8) sich einschalten und ausschalten lässt,
wobei die eingeschaltete Fluidfördereinheit (8) dazu ausgestaltet ist, die Gasprobe (Gp) aus der Messkammer (2) heraus und durch die Abführ-Fluidführungseinheit (4, 15) hindurch zu fördern, und
wobei der Drucksensor (27) dazu ausgestaltet ist, den Druck in der Abführ-Fluidführungseinheit (4, 15) zu messen, und
wobei das Gasmessgerät (100) zusätzlich dazu ausgestaltet ist, abhängig vom gemessenen Druck
- zu entscheiden, ob die Fluidfördereinheit (8) eingeschaltet oder ausgeschaltet ist, und / oder
- einen von der Fluidfördereinheit (8) erzielten Volumenfluss zu messen.

7. Gasmessgerät (100) nach einem der vorhergehenden Ansprüche,
**dadurch gekennzeichnet, dass**
das Gasmessgerät (100) eine Fluidfördereinheit (8) umfasst,
wobei die Fluidfördereinheit (8)
- eine Kammer (17) mit einem veränderbaren Volumen,
- einen Stellantrieb (28),
- ein Ansaug-Rückschlagventil (31) und
- ein Ausstoß-Rückschlagventil (32)
umfasst,
wobei der Stellantrieb (28) dazu ausgestaltet ist, das Volumen der Kammer (17) zu verändern,
wobei die Kammer (17) der Fluidfördereinheit (8) in einer Ansaug-Fluidverbindung (9, 10) mit der Messkammer (2) und in einer Ausstoß-Fluidverbindung (12) mit der Abführ-Fluidführungseinheit (4, 15) steht,
wobei das Ansaug-Rückschlagventil (31) dazu ausgestaltet ist,
- einen Fluss von Fluid durch die Ansaug-Fluidverbindung (9, 10) hindurch in die Kammer (17) zu ermöglichen und
- einen Fluss in die entgegengesetzte Richtung zu unterbinden, und
wobei das Ausstoß-Rückschlagventil (32) dazu ausgestaltet ist,
- einen Fluss von Fluid aus der Kammer (17) in die Ausstoß-Fluidverbindung (12) hinein zu ermöglichen und
- einen Fluss in die entgegengesetzte Richtung zu unterbinden.

8. Gasmessgerät (100) nach einem der vorhergehenden Ansprüche,
**dadurch gekennzeichnet, dass**
der oder ein Gassensor (50) eine Strahlungsquelle (35) und einen Photodetektor (36) umfasst,
wobei die Strahlungsquelle (35) dazu ausgestaltet ist, elektromagnetische Strahlung zu emittieren,
wobei das Gasmessgerät (100) so ausgestaltet ist, dass emittierte Strahlung mindestens einmal die Messkammer (2) durchdringt und auf den Photodetektor (36) auftrifft, und
wobei der Photodetektor (36) dazu ausgestaltet ist,
abhängig von der Intensität von auftreffender Strahlung dergestalt ein Signal zu generieren,
dass das generierte Signal eine Information über die Konzentration des Bestandteils der Gasprobe (Gp) umfasst.

9. Gasmessgerät (100) nach Anspruch 8,
**dadurch gekennzeichnet, dass**
das Gasmessgerät (100) einen weiteren Gassensor (38) umfasst, der eine elektrische Detektionsgröße aufweist,
wobei das Gasmessgerät (100)
- in einer Alternative dazu ausgestaltet ist, dass die abgezweigte Gasprobe (Gp) sowohl den Gassensor (50) mit der Strahlungsquelle (35) als auch den weiteren Gassensor (38) erreicht, und
- in einer anderen Alternative dazu ausgestaltet ist, dass eine weitere Gasprobe aus dem durchfließenden Gasgemisch (A) abgezweigt wird und den weiteren Gassensor (38) erreicht,
wobei der weitere Gassensor (38) dazu ausgestaltet ist, dass im weiteren Gassensor (38) eine chemische Reaktion abläuft und die chemische Reaktion die elektrische Detektionsgröße beeinflusst,
wobei die chemische Reaktion und damit die elektrische Detektionsgröße von der Konzentration des Bestandteils in der Gasprobe (Gp) oder in der weiteren Gasprobe abhängen und
wobei der weitere Gassensor (38) dazu ausgestaltet ist,
- die elektrische Detektionsgröße zu messen und
- abhängig von der gemessenen elektrischen Detektionsgröße dergestalt ein Signal zu generieren, dass das generierte Signal eine Information über die Konzentration des Bestandteils in der Gasprobe (Gp) oder in der weiteren Gasprobe umfasst.

10. Gasmessverfahren zum Messen der Konzentration mindestens eines Bestandteils in einem Gasgemisch (A),
unter Verwendung eines Gasmessgeräts (100), das
- eine röhrenförmige Eingabeeinheit (1),
- einen Grundkörper (6, 16),
- eine Zuführ-Fluidführungseinheit (3, 39),
- eine Messkammer (2),
- eine Abführ-Fluidführungseinheit (4, 15) und
- mindestens einen Gassensor (50, 38)
umfasst,
wobei die Eingabeeinheit (1)
- eine Längsachse (L.1), eine Einlassöffnung (Ö.e) und eine Auslassöffnung (Ö.a) aufweist und
- wenigstens zeitweise mit dem Grundkörper (6, 16) verbunden ist,
wobei die Messkammer (2) im Inneren des Grundkörpers (6, 16) angeordnet ist,
wobei die beiden Fluidführungseinheiten (3, 39; 4, 15) voneinander beabstandet im Grundkörper (6, 16) angeordnet sind,
wobei das Gasmessverfahren die Schritte umfasst, dass
- das Gasgemisch (A) durch die Einlassöffnung (Ö.e) in die Eingabeeinheit (1) hinein und durch die Eingabeeinheit (1) hindurch fließt,
- eine Gasprobe (Gp) in einem Eintrittspunkt (P.e) im Grundkörper (6, 16) aus dem durchfließenden Gasgemisch (A) abgezweigt wird und
- der übrige Teil des Gasgemischs (A), also das Gasgemisch (A) ohne die abgezweigte Gasprobe (Gp), am Eintrittspunkt (P.e) vorbei fließt,
wobei das Gasmessverfahren die weiteren Schritte umfasst, dass
- die abgezweigte Gasprobe (Gp) vom Eintrittspunkt (P.e) durch die Zuführ-Fluidführungseinheit (3, 39) hindurch in die Messkammer (2) fließt,
- der oder jeder Gassensor (50, 38) jeweils die Konzentration des Bestandteils in der Gasprobe (Gp) in der Messkammer (2) misst und
- die Gasprobe (Gp) aus der Messkammer (2) durch die Abführ-Fluidführungseinheit (4, 15) hindurch zum Austrittspunkt (P.a1) fließt, und
wobei das Gasmessverfahren die weiteren Schritte umfasst, dass
- die abgezweigte Gasprobe (Gp) im Austrittspunkt (P.a1) aus dem Grundkörper (6, 16) heraus und an der Eingabeeinheit (1) vorbei fließt und
- sowohl die abgezweigte Gasprobe (Gp) als auch der übrige Teil des Gasgemischs (A) an zwei voneinander beabstandeten Stellen in eine Umgebung des Gasmessgeräts (100) fließen,
**dadurch gekennzeichnet, dass**
das Gasmessgerät (100) so ausgestaltet ist, dass dann, wenn die Eingabeeinheit (1) mit dem Grundkörper (6, 16) verbunden ist, ein Kanal (25) zwischen dem Grundkörper (6, 16) und der Eingabeeinheit (1) auftritt,
wobei dieser Kanal (25) den Austrittspunkt (P.a1) mit einer Umgebung des Gasmessgeräts (100) verbindet und sich parallel zur Längsachse (L.1) der Eingabeeinheit (1) erstreckt, und
die abgezweigte Gasprobe (Gp) beim Fließen an der Eingabeeinheit (1) vorbei in eine Richtung weg von der Einlassöffnung (Ö.e) und dabei vom Austrittspunkt (P.a1) durch den Kanal (25) hindurch fließt.

## Claims

1. Gas-measuring device (100) for measuring the concentration of at least one component in a gas mixture (A),
the gas-measuring device (100) comprising
- a tubular input unit (1),
- a main body (6, 16),
- a supply fluid guide unit (3, 39),
- a measuring chamber (2),
- a discharge fluid guide unit (4, 15) and
- at least one gas sensor (50, 38),
the input unit (1)
- having a longitudinal axis (L.1), an inlet opening (Ö.e) and an outlet opening (Ö.a) and
- being connected or connectable to the main body (6, 16),
the measuring chamber (2) being arranged inside the main body (6, 16),
the supply fluid guide unit (3, 39) connecting an entry point (P.e) in the main body (6, 16) to the measuring chamber (2) and the discharge fluid guide unit (4, 15) connecting the measuring chamber (2) to an exit point (P.a1) in the main body (6, 16),
the two fluid guide units (3, 39; 4, 15) being spaced apart from one another,
the gas-measuring device (100) being configured such that
- a gas mixture (A) flows through the inlet opening (Ö.e) into the input unit (1) and through the input unit (1),
- a gas sample (Gp) is diverted from the flowing gas mixture (A) at the entry point (P.e),
- the remaining portion of the gas mixture (A), i.e. the gas mixture (A) without the diverted gas sample (Gp), flows past the entry point (P.e),
the gas-measuring device (100) being further configured such that the diverted gas sample (Gp)
- flows from the entry point (P.e) through the supply fluid guide unit (3, 39) into the measuring chamber (2) and
- flows from the measuring chamber (2) through the discharge fluid guide unit (4, 15) to the exit point (P.a1), and
the gas-measuring device (100) being further configured such that
- the diverted gas sample (Gp) flows out of the main body (6, 16) at the exit point (P.a1) and past the input unit (1) and
- the diverted gas sample (Gp) and the remaining portion of the gas mixture (A) flow into an environment of the gas-measuring device (100) at two points which are spaced apart from one another, and
the or each gas sensor (50, 38) is configured to measure the concentration of the component in the gas sample (Gp) in the measuring chamber (2) in each case,
**characterized in that**
the gas-measuring device (100) is configured such that, if the input unit (1) is connected to the main body (6, 16), a channel (25) occurs between the main body (6, 16) and the input unit (1),
this channel (25) connecting the exit point (P.a1) to an environment of the gas-measuring device (100) and extending in parallel with the longitudinal axis (L.1) of the input unit (1), and
the gas-measuring device (100) being further configured such that the diverted gas sample (Gp) flows past the input unit (1) in a direction away from the inlet opening (Ö.e) and through the channel (25) from the exit point (P.a1).

2. Gas-measuring device (100) according to claim 1,
**characterized in that**
the main body (6, 16), viewed in a flow direction of the gas mixture (A) through the input unit (1), protrudes downstream from the input unit (1) over the input unit (1)
so that, downstream of the outlet opening (Ö.a), a region (Vb) is formed which is delimited on one side by the input unit (1) and on the other side by the main body (6, 16).

3. Gas-measuring device (100) according to either of the preceding claims,
**characterized in that**
a separating element (24) divides the input unit (1) into an inlet-side chamber (K.e) and an outlet-side chamber (K.a),
the inlet opening (Ö.e) being adjacent to the inlet-side chamber (K.e),
the outlet opening (Ö.a) being adjacent to the outlet-side chamber (K.a),
at least one through-opening (Ö.24) in the separating element (24) connecting the two chambers (K.e, K.a) to one another, and
both the entry point (P.e) and the exit point (P.a1) being adjacent to the outlet-side chamber (K.a).

4. Gas-measuring device (100) according to claim 3,
**characterized in that**
the inlet-side chamber (K.e) connects the inlet opening (Ö.e) to the outlet opening (Ö.a) and,
viewed in a flow direction of the gas mixture (A) through the input unit (1), a segment of the inlet-side chamber (K.e) is located next to the outlet-side chamber (K.a).

5. Gas-measuring device (100) according to any of the preceding claims,
**characterized in that**
the input unit (1) is detachably connected or connectable to the main body (6, 16) and
the gas-measuring device (100) comprises a pressure sensor (27),
the pressure sensor (27) being configured to measure the pressure in the supply fluid guide unit (3, 39) or the pressure in the discharge fluid guide unit (4, 15), and
the gas-measuring device (100) being configured to automatically decide, depending on the measured pressure, whether the input unit (1) is connected to the main body (6, 16) or not.

6. Gas-measuring device (100) according to any of the preceding claims,
**characterized in that**
the gas-measuring device (100) comprises a pressure sensor (27) and a fluid-conveying unit (8),
it being possible to switch the fluid-conveying unit (8) on and off,
the switched-on fluid-conveying unit (8) being configured to convey the gas sample (Gp) out of the measuring chamber (2) and through the discharge fluid guide unit (4, 15), and
the pressure sensor (27) being configured to measure the pressure in the discharge fluid guide unit (4, 15), and
the gas-measuring device (100) being additionally configured, depending on the measured pressure,
- to decide whether the fluid-conveying unit (8) is switched on or off, and/or
- to measure a volume flow achieved by the fluid-conveying unit (8).

7. Gas-measuring device (100) according to any of the preceding claims,
**characterized in that**
the gas-measuring device (100) comprises a fluid-conveying unit (8),
the fluid-conveying unit (8) comprising
- a chamber (17) having a parameter volume,
- an actuator (28),
- an intake check valve (31) and
- an output check valve (32),
the actuator (28) being configured to change the volume of the chamber (17),
the chamber (17) of the fluid-conveying unit (8) being in an intake fluid connection (9, 10) with the measuring chamber (2) and in an output fluid connection (12) with the discharge fluid guide unit (4, 15),
the intake check valve (31) being configured
- to allow a flow of fluid through the intake fluid connection (9, 10) into the chamber (17) and
- to stop a flow in the opposite direction, and
the output check valve (32) being configured
- to allow a flow of fluid from the chamber (17) into the output fluid connection (12) and
- to stop a flow in the opposite direction.

8. Gas-measuring device (100) according to any of the preceding claims,
**characterized in that**
the or a gas sensor (50) comprises a radiation source (35) and a photodetector (36),
the radiation source (35) being configured to emit electromagnetic radiation,
the gas-measuring device (100) being configured such that the emitted radiation passes through the measuring chamber (2) at least once and impinges on the photodetector (36), and
the photodetector (36) being configured to generate
a signal depending on the intensity of incident radiation such
that the generated signal comprises information about the concentration of the component of the gas sample (Gp).

9. Gas-measuring device (100) according to claim 8,
**characterized in that**
the gas-measuring device (100) comprises a further gas sensor (38) which has an electrical detection parameter,
the gas-measuring device (100) being configured
- in an alternative configuration such that the diverted gas sample (Gp) reaches the gas sensor (50) with the radiation source (35) and reaches the further gas sensor (38), and
- in another alternative configuration such that a further gas sample is diverted from the flowing gas mixture (A) and reaches the further gas sensor (38),
the further gas sensor (38) being configured such that a chemical reaction takes place in the further gas sensor (38) and the chemical reaction influences the electrical detection parameter,
the chemical reaction and thus the electrical detection parameter depending on the concentration of the component in the gas sample (Gp) or in the further gas sample, and
the further gas sensor (38) being configured
- to measure the electrical detection parameter and,
- depending on the measured electrical detection parameter, to generate a signal such that the generated signal includes information about the concentration of the component in the gas sample (Gp) or in the further gas sample.

10. Gas measuring method for measuring the concentration of at least one component in a gas mixture (A)
using a gas-measuring device (100) which comprises
- a tubular input unit (1),
- a main body (6, 16),
- a supply fluid guide unit (3, 39),
- a measuring chamber (2),
- a discharge fluid guide unit (4, 15) and
- at least one gas sensor (50, 38),
the input unit (1)
- having a longitudinal axis (L.1), an inlet opening (Ö.e) and an outlet opening (Ö.a) and
- being connected at least temporarily to the main body (6, 16),
the measuring chamber (2) being arranged inside the main body (6, 16),
the two fluid guide units (3, 39; 4, 15) being arranged spaced apart from one another in the main body (6, 16),
the gas measuring method comprising the steps following steps
- the gas mixture (A) flows through the inlet opening (Ö.e) into the input unit (1) and through the input unit (1),
- a gas sample (Gp) is diverted from the flowing gas mixture (A) at an entry point (P.e) in the main body (6, 16) and
- the remaining portion of the gas mixture (A), i.e. the gas mixture (A) without the diverted gas sample (Gp), flows past the entry point (P.e),
the gas measuring method comprising the following further steps
- the diverted gas sample (Gp) flows from the entry point (P.e) through the supply fluid guide unit (3, 39) into the measuring chamber (2),
- the or each gas sensor (50, 38) measures the concentration of the component in the gas sample (Gp) in the measuring chamber (2) and
- the gas sample (Gp) flows from the measuring chamber (2) through the discharge fluid guide unit (4, 15) to the exit point (P.a1), and
the gas measuring method comprising the following further steps
- the diverted gas sample (Gp) flows out of the main body (6, 16) at the exit point (P.a1) and past the input unit (1) and
- the diverted gas sample (Gp) and the remaining portion of the gas mixture (A) flow into an environment of the gas-measuring device (100) at two points which are spaced apart from one another,
**characterized in that**
the gas-measuring device (100) is configured such that if the input unit (1) is connected to the main body (6, 16), a channel (25) occurs between the main body (6, 16) and the input unit (1),
this channel (25) connecting the exit point (P.a1) to an environment of the gas-measuring device (100) and extending in parallel with the longitudinal axis (L.1) of the input unit (1), and
the diverted gas sample (Gp) flows past the input unit (1) in a direction away from the inlet opening (Ö.e) and through the channel (25) from the exit point (P.a1).

## Revendications

1. Appareil de mesure de gaz (100) destiné à mesurer la concentration d'au moins un composant dans un mélange gazeux (A),
dans lequel l'appareil de mesure à gaz (100) comprend
- une unité d'admission (1) de forme tubulaire,
- un corps de base (6, 16),
- une unité de guidage de fluide d'alimentation (3, 39),
- une chambre de mesure (2),
- une unité de guidage de fluide de refoulement (4, 15) et
- au moins un capteur de gaz (50, 38),
dans lequel l'unité d'admission (1)
- présente un axe longitudinal (L.1), une ouverture d'admission (Ö.e) et une ouverture d'évacuation (Ö.a), et
- est reliée ou peut être reliée au corps de base (6, 16),
dans lequel la chambre de mesure (2) est agencée à l'intérieur du corps de base (6, 16),
dans lequel l'unité de guidage de fluide d'alimentation (3, 39) relie un point d'entrée (P.e) dans le corps de base (6, 16) à la chambre de mesure (2) et l'unité de guidage de fluide de refoulement (4, 15) relie la chambre de mesure (2) à un point de sortie (P.a1) dans le corps de base (6, 16),
dans lequel les deux unités de guidage de fluide (3, 39 ; 4, 15) sont espacées l'une de l'autre,
dans lequel l'appareil de mesure de gaz (100) est configuré de sorte que
- un mélange gazeux (A) s'écoule dans l'unité d'admission (1) par l'ouverture d'admission (Ö.e) et traverse l'unité d'admission (1),
- un échantillon de gaz (Gp) est prélevé au point d'entrée (P.e) à partir du mélange gazeux (A) qui s'écoule,
- la partie restante du mélange gazeux (A), c'est-à-dire le mélange gazeux (A) sans l'échantillon de gaz (Gp) prélevé, s'écoule au-delà du point d'entrée (P.e),
dans lequel l'appareil de mesure de gaz (100) est en outre configuré de telle sorte que l'échantillon de gaz (Gp) prélevé
- s'écoule depuis le point d'entrée (P.e), par l'unité de guidage de fluide d'alimentation (3, 39), vers la chambre de mesure (2) et
- s'écoule depuis la chambre de mesure (2) par l'unité de guidage de fluide de refoulement (4, 15) jusqu'au point de sortie (P.a1), et
dans lequel l'appareil de mesure de gaz (100) est en outre configuré de sorte que
- l'échantillon de gaz (Gp) prélevé s'écoule au point de sortie (P.a1) hors du corps de base (6, 16) et au-delà de l'unité d'admission (1), et
- tant l'échantillon de gaz (Gp) prélevé que la partie restante du mélange gazeux (A) s'écoulent vers l'environnement de l'appareil de mesure de gaz (100) en deux points espacés l'un de l'autre, et
dans lequel le ou chaque capteur de gaz (50, 38) est configuré pour mesurer respectivement la concentration du composant dans l'échantillon de gaz (Gp) présent dans la chambre de mesure (2),
**caractérisé en ce que**
l'appareil de mesure de gaz (100) est configuré de telle sorte que, lorsque l'unité d'admission (1) est reliée au corps de base (6, 16), un canal (25) se forme entre le corps de base (6, 16) et l'unité d'admission (1),
dans lequel le canal (25) relie le point de sortie (P.a1) à l'environnement de l'appareil de mesure de gaz (100) et s'étend parallèlement à l'axe longitudinal (L.1) de l'unité d'admission (1) et
dans lequel l'appareil de mesure de gaz (100) est en outre configuré de sorte que
l'échantillon de gaz (Gp) prélevé, lorsqu'il s'écoule le long de l'unité d'admission (1), s'éloigne de l'ouverture d'admission (Ö.e) et passe ainsi du point de sortie (P.a1) par le canal (25).

2. Appareil de mesure de gaz (100) selon la revendication 1,
**caractérisé en ce que**
le corps de base (6, 16) - vu dans un sens d'écoulement du mélange gazeux (A) par l'unité d'admission (1) - dépasse de l'unité d'admission (1) en aval de celle-ci, en passant par l'unité d'admission (1),
de sorte qu'en aval de l'ouverture d'évacuation (Ö.a), il se forme une zone (Vb) qui est délimitée d'un côté par l'unité d'admission (1) et de l'autre côté par le corps de base (6, 16).

3. Appareil de mesure de gaz (100) selon l'une des revendications précédentes,
**caractérisé en ce que**
un élément de séparation (24) divise l'unité d'admission (1) en une chambre côté admission (K.e) et une chambre côté évacuation (K.a),
dans lequel l'ouverture d'admission (Ö.e) est adjacente à la chambre côté admission (K.e),
dans lequel l'ouverture d'évacuation (Ö.a) est adjacente à la chambre côté évacuation (K.a),
dans lequel au moins une ouverture de passage (Ö.24) dans l'élément de séparation (24) relie entre elles les deux chambres (K.e, K.a) et
dans lequel tant le point d'entrée (P.e) que le point de sortie (P.a1) sont adjacents à la chambre côté évacuation (K.a).

4. Appareil de mesure de gaz (100) selon la revendication 3,
**caractérisé en ce que**
la chambre côté admission (K.e) relie l'ouverture d'admission (Ö.e) à l'ouverture d'évacuation (Ö.a) et
vu dans un sens d'écoulement du mélange gazeux (A) par l'unité d'admission (1), un segment de la chambre côté admission (K.e) se trouve à côté de la chambre côté évacuation (K.a).

5. Appareil de mesure de gaz (100) selon l'une des revendications précédentes,
**caractérisé en ce que**
l'unité d'admission (1) est reliée ou peut être reliée de manière amovible au corps de base (6, 16) et
l'appareil de mesure de gaz (100) comprend un capteur de position (27),
dans lequel le capteur de pression (27) est configuré pour mesurer la pression dans l'unité de guidage de fluide d'alimentation (3, 39) ou la pression dans l'unité de guidage du fluide de refoulement (4, 15), et
dans lequel l'appareil de mesure de gaz (100) est configuré pour décider automatiquement, en fonction de la pression mesurée, si l'unité d'admission (1) est reliée ou non au corps de base (6, 16).

6. Appareil de mesure de gaz (100) selon l'une des revendications précédentes,
**caractérisé en ce que**
l'appareil de mesure de gaz (100) comprend un capteur de pression (27) et une unité de transport de fluide (8),
dans lequel l'unité de transport de fluide (8) peut être mise en marche et arrêtée,
dans lequel l'unité de transport de fluide (8), lorsqu'elle est activée, est configurée pour transporter l'échantillon de gaz (Gp) hors de la chambre de mesure (2) et à travers l'unité de guidage de fluide de refoulement (4, 15), et
dans lequel le capteur de pression (27) est configuré pour mesurer la pression dans l'unité de guidage de fluide de refoulement (4, 15), et
dans lequel l'appareil de mesure de gaz (100) est en outre configuré pour, en fonction de la pression mesurée
- décider si l'unité de transport de fluide (8) est en marche ou arrêtée, et/ou
- mesurer un débit volumique généré par l'unité de transport de fluide (8).

7. Appareil de mesure de gaz (100) selon l'une des revendications précédentes,
**caractérisé en ce que**
le système de mesure de gaz (100) comprend une unité de transport de fluide (8),
dans lequel l'unité de transport de fluide (8) comprend
- une chambre (17) comportant un volume variable,
- un organe de réglage (28),
- un clapet anti-retour d'aspiration (31) et
- un clapet anti-retour de délivrance (32),
dans lequel l'organe de réglage (28) est configuré pour modifier le volume de la chambre (17),
dans lequel la chambre (17) de l'unité de transport de fluide (8) est en liaison fluidique d'aspiration (9, 10) avec la chambre de mesure (2) et est en liaison fluidique de délivrance (12) avec l'unité de guidage de fluide de refoulement (4, 15),
dans lequel le clapet anti-retour d'aspiration (31) est configuré,
- pour permettre à un écoulement de fluide de passer par la liaison fluidique d'aspiration (9, 10) pour entrer dans la chambre (17) et
- pour empêcher un écoulement dans le sens opposé, et
dans lequel le clapet anti-retour de délivrance (32) est configuré,
- pour permettre à un écoulement de fluide de passer par la chambre (17) pour entrer dans la liaison fluidique de délivrance (12) et
- pour empêcher un écoulement dans le sens opposé.

8. Appareil de mesure de gaz (100) selon l'une des revendications précédentes,
**caractérisé en ce que**
le ou un capteur de gaz (50) comprend une source de rayonnement (35) et un photodétecteur (36),
dans lequel la source de rayonnement (35) est configurée pour émettre un rayonnement électromagnétique,
dans lequel l'appareil de mesure de gaz (100) est configuré de telle sorte que le rayonnement émis traverse au moins une fois la chambre de mesure (2) et atteigne le photodétecteur (36), et
dans lequel le photodétecteur (36) est configuré pour générer un signal en fonction de l'intensité du rayonnement incident, de telle sorte que le signal généré comprend une information sur la concentration du composant de l'échantillon de gaz (Gp).

9. Appareil de mesure de gaz (100) selon la revendication 8,
**caractérisé en ce que**
l'appareil de mesure de gaz (100) comprend un autre capteur de gaz (38) qui présente une grandeur de détection électrique,
dans lequel l'appareil de mesure à gaz (100)
- est configuré, dans une variante, de telle sorte que l'échantillon de gaz (Gp) prélevé atteint à la fois le capteur de gaz (50) comportant la source de rayonnement (35) et l'autre capteur de gaz (38), et
- est configuré, dans une autre variante, de telle sorte qu'un autre échantillon de gaz est prélevé du mélange gazeux (A) qui s'écoule et atteint l'autre capteur de gaz (38),
dans lequel l'autre capteur de gaz (38) est configuré de telle sorte qu'une réaction chimique se produit à l'intérieur de l'autre capteur de gaz (38) et que la réaction chimique influence la grandeur de détection électrique,
dans lequel la réaction chimique, et donc la grandeur de détection électrique, dépendent de la concentration du composant dans l'échantillon de gaz (Gp) ou dans l'autre échantillon de gaz, et
dans lequel l'autre capteur de gaz (38) est configuré,
- pour mesurer la grandeur de détection électrique et
- pour générer un signal en fonction de la grandeur de détection électrique mesurée, de telle sorte que le signal généré comprend une information sur la concentration du composant dans l'échantillon de gaz (Gp) ou dans l'autre échantillon de gaz.

10. Procédé de mesure de gaz destiné à mesurer la concentration d'au moins un composant dans un mélange gazeux (A), à l'aide d'un appareil de mesure de gaz (100) qui comprend
- une unité d'admission (1) de forme tubulaire,
- un corps de base (6, 16),
- une unité de guidage de fluide d'alimentation (3, 39),
- une chambre de mesure (2),
- une unité de guidage de fluide de refoulement (4, 15) et
- au moins un capteur de gaz (50, 38),
dans lequel l'unité d'admission (1)
- présente un axe longitudinal (L.1), une ouverture d'admission (Ö.e) et une ouverture d'évacuation (Ö.a), et
- est reliée, au moins temporairement, au corps de base (6, 16),
dans lequel la chambre de mesure (2) est agencée à l'intérieur du corps de base (6, 16),
dans lequel les deux unités de guidage de fluide (3, 39 ; 4, 15) sont agencées espacées l'une de l'autre dans le corps de base (6, 16),
dans lequel le dispositif de mesure de gaz comprend les étapes selon lesquelles
- le mélange gazeux (A) s'écoule dans l'unité d'admission (1) par l'ouverture d'admission (Ö.e) et traverse l'unité d'admission (1),
- un échantillon de gaz (Gp) est prélevé en un point d'entrée (P.e) dans le corps de base (6, 16) à partir du mélange gazeux (A) qui s'écoule, et
- la partie restante du mélange gazeux (A), c'est-à-dire le mélange gazeux (A) sans l'échantillon de gaz (Gp) prélevé, s'écoule au-delà du point d'entrée (P.e),
dans lequel le dispositif de mesure de gaz comprend les étapes supplémentaires selon lesquelles
- l'échantillon de gaz (Gp) prélevé s'écoule depuis le point d'entrée (P.e), par l'unité de guidage de fluide d'alimentation (3, 39), vers la chambre de mesure (2),
- le ou chaque capteur de gaz (50, 38) mesure respectivement la concentration du composant dans l'échantillon de gaz (Gp) dans la chambre de mesure (2) et
- l'échantillon de gaz (Gp) s'écoule depuis la chambre de mesure (2) par l'unité de guidage de fluide de refoulement (4, 15) jusqu'au point de sortie (P.a1), et
dans lequel le dispositif de mesure de gaz comprend les étapes supplémentaires selon lesquelles
- l'échantillon de gaz (Gp) prélevé s'écoule au point de sortie (P.a1) hors du corps de base (6, 16) et au-delà de l'unité d'admission (1), et
- tant l'échantillon de gaz (Gp) prélevé que la partie restante du mélange gazeux (A) s'écoulent vers l'environnement de l'appareil de mesure de gaz (100) en deux points espacés l'un de l'autre,
**caractérisé en ce que**
l'appareil de mesure de gaz (100) est configuré de telle sorte que, lorsque l'unité d'admission (1) est reliée au corps de base (6, 16), un canal (25) se forme entre le corps de base (6, 16) et l'unité d'admission (1),
dans lequel le canal (25) relie le point de sortie (P.a1) à l'environnement de l'appareil de mesure de gaz (100) et s'étend parallèlement à l'axe longitudinal (L.1) de l'unité d'admission (1), et
l'échantillon de gaz (Gp) prélevé, lorsqu'il s'écoule le long de l'unité d'admission (1), s'éloigne de l'ouverture d'admission (Ö.e) et passe ainsi du point de sortie (P.a1) par le canal (25).
